(19) Europäisches Patentamt
European Patent Office
Office européen des brevets

(11) Publication number: **0 233 376 B1**

(12) **EUROPEAN PATENT SPECIFICATION**

(45) Date of publication of patent specification: **22.01.92**

(51) Int. Cl.[5]: **C08L 27/06**, C08L 75/04, C08K 5/10, A61L 29/00, A61L 31/00

(21) Application number: **86200220.1**

(22) Date of filing: **17.02.86**

(54) **Radiopaque thermoplastic resin-silicone network resin compositions and medical-surgical tubings prepared therefrom.**

(43) Date of publication of application:
**26.08.87 Bulletin 87/35**

(45) Publication of the grant of the patent:
**22.01.92 Bulletin 92/04**

(84) Designated Contracting States:
**DE FR GB IT NL**

(56) References cited:
**US-A- 3 645 955**
**US-A- 4 250 072**
**US-A- 4 500 688**

(73) Proprietor: **BECTON, DICKINSON & COMPANY**
**One Becton Drive**
**Franklin Lakes New Jersey 07417-1880(US)**

(72) Inventor: **Flynn, Vincent J.**
**130 New Road, Apt. D-10**
**Parsippany, NJ 07054(US)**

(74) Representative: **Roggero, Sergio et al**
**Ing. Barzanò & Zanardo Milano S.p.A. Via Borgonuovo 10**
**I-20121 Milano(IT)**

**EP 0 233 376 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel radiopaque resin compositions, and to medical-surgical tubings prepared therefrom. More particularly, the invention pertains to compositions of polyiodobenzoic acid ester-containing resins, alone, or combinations of such resins, and a platinum-cured silicone network polymer dispersed therein and their use in medical-surgical devices, especially tubing and catheters.

### BACKGROUND OF THE INVENTION

Archer and Flynn, U.S. Patent No. 3,361,700, disclose that a family of alkoxyalkyl esters of diiodobenzoic acid are radiopaque and suitable to plasticize vinyl resins into a form useful to manufacture tubings for catheters and similar products. Flynn, U.S. Patent No. 3,645,955 discloses that di- and tetraiodoesters used alone or in combination with the alkoxyalkyl diiodoesters are superior for this purpose because they show less tendency to exude and lower concentrations provide a better balance between flexibility and stiffness. United Kingdom Patent Specification No. 866,184 discloses a family of esters of triiodobenzoic acid. They have been found to be useful to plasticize resins while at the same time rendering them radiopaque. Burlis et al, U.S. Patent No. 3,752,617 disclose methods for making multiwall tubing, co-tapered along its length, but use plastics other than polyurethane, and make no mention of any additives, specifically by name, to produce different X-ray sensitive characteristics. It has also been proposed to provide thermoplastic, e.g., polyurethane, tubing rendered non-thrombogenic with a coating layer of silicone polymer.

While the iodoester opacified vinyl resin compositions of U.S. 3,361,700 are quite suitable for the production of tubing of simple types useful for intubation sets and catheter needles, they are not completely satisfactory for production of shaped devices. For example, if flared, or if formed into curved tips, the shapes tend to revert to straight tubing - a so-called loss of plastic memory effect. In applicant's U.S. Patents Numbers 4,250,072 and 4,283,447, it is disclosed that if the vinyl resin is replaced partially or completely by thermoplastic polyurethane, the iodoester radiopacified compositions are amenable to the induction of complex shapes - and they'll stay that way. Such tubings provide catheters eminently suitable for selective renal arteriography and for celial arteriography, and also for the manufacture of pig-tail catheters. Surprisingly, the iodoesters do not show the same tendency to overplasticize the urethane esters as they do the vinyl esters, and, moreover, there is lesser need to use a radioparent plasticizer with urethanes and, in fact, it is preferred to omit a radioparent plasticizer completely. As is shown therein, the compositions lend themselves well to the formation of highly advantageous multi-wall tubing constructions. In one feature of the said patents, a thin, inner core of iodoester-radiopacified polyurethane and a thicker outer core of plasticized vinyl resin eliminates extraction problems with fluids passing through the core. In another feature, an inner core of nylon or polypropylene provides an outer jacket of iodoester radiopacified urethane or urethane-vinyl with stiffness, but much less tendency to kink in a multiwall construction; and such tubes are also easily manufactured in a co-tapered wall thickness construction in which the variations in twisting resistance provide enhances ease of insertion through torturous body passages. A surface coating of silicone resin helps improve ease of insertion when properly formulated, while reducing thrombogenicity, but such constructions still can be improved, especially in terms of heat resistance.

More particularly, it is desirable to improve such structures in the following ways: to provide a more hydrophobic surface which allows fluid levels to be read correctly; to provide a surface with very little and, preferably, no filler exposed to minimize dangerous thrombogenic response; to provide a surface with reduced friction and wear; and to provide tubings and catheters made from such tubings which are more heat resistant and accordingly have improved sterilizability and service life.

It has now been found, and is the subject matter of the present invention, that if controlled amounts of platinum-cured silicone network polymers are employed in such compositions, tubings and catheters made therefrom will provide the above-enumerated advantages, and many others.

### BRIEF DESCRIPTION OF THE DRAWINGS

The invention will be better understood by reference to the accompany drawings in which:

FIGURE 1 is a longitudinal view of a catheter made from one form of the tubing made in accordance with the present invention and wherein, for illustrative purposes, the distal end is tapered and shaped to form a "J" tip, and the proximal end is flared;

FIGURE 2 is an enlarged cross-sectional view of radiopaque tubing according to this invention;

FIGURE 3 is an elongated cross-sectional view of tubing having a radiopaque inner core and a transparent outer shell;

FIGURE 4 is an enlarged cross-sectional view of tubing having a transparent core and a radiopaque outer shell; and

FIGURE 5 is an enlarged, fragmentary cross-sectional view along the longitudinal axis of an embodiment tube of this invention having a transparent co-tapered core and a radiopaque, correspondingly co-tapered jacket, extruded as in FIGURE 6.

## DESCRIPTION OF THE INVENTION

In accordance with the present invention, there are provided radiopaque compositions comprising:

(a) a resin which includes from 100 to 0 parts by weight of a thermoplastic polyurethane and correspondingly from 0 to 100 parts of a polymer or copolymer of a halogenated vinyl monomer per 100 parts by weight of resin;

(b) a radiopacifier therefor consisting of a diiodobenzoate or a tetraiodobenzoate of formula (I) or a triiodobenzoate of formula (II)

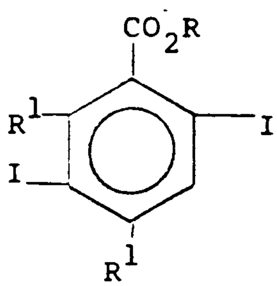

(I)

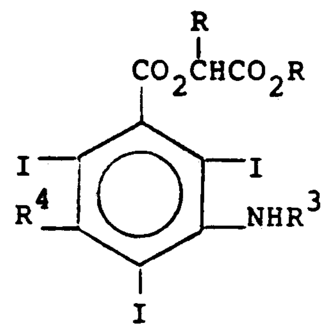

(II)

wherein R is alkyl or alkoxy-alkyl, $R^1$ is hydrogen or iodo, $R^2$ is hydrogen or alkyl, $R^3$ is hydrogen or alkanoyl, $R^4$ is hydrogen or alkanoylamino, and $R^5$ is alkyl, or a mixture of said compounds, said radiopacifier comprising 10 to 40 parts by weight per 100 parts by weight of (a) and (b), and, uniformly dispersed therein;

(c) a platinum-cured silicone network polymer, the amount of (c) comprising 8 to 30 parts by weight per 100 parts by weight of (a), (b) and (c), wherein said radiopacifier composition is essentially free of filler.

The carbon content of radicals representing R, $R^1$, $R^2$, $R^3$, $R^4$ and $R^5$ can range from 1 to 30, but preferably it will range from 1 to 6 carbon atoms.

Preferably, in component (b)(I), each $R^1$ is iodo and R is (loweralkyl or (lower)alkoxy (lower)alkyl. The most preferred radiopacifiers of formula (I) are n-butyl 2,3,4,6-tetraiodobenzoate, 2-ethoxyethyl 2,5-diiodobenzoate, or a mixture thereof. The most preferred radiopacifiers of formula (II) are ethyl (3-amino-2,4,6-triiodobenzoyloxy) acetate and ethyl 2-(3-amino-2,4,6-triiodobenzoyloxy) butanoate.

In preferred embodiments resin component (a) comprises from 100 to 33 parts by weight of thermoplastic polyurethane and correspondingly from 0 to 67 parts by weight of a polymer of a halogenated vinyl monomer per 100 parts by weight of resin. Preferred also are compositions wherein resin component (a) comprises a vinyl resin which is a polymer or copolymer of a halogenated vinyl monomer.

Preferably, component (c) comprises the reaction product of

(i) an organic silicon compound having at least two silicon-bonded unsaturated groups per molecule,

(ii) a silicon compound contaiing at least two silicon-bonded hydrogen atoms per molecule, and

(iii) a small catalytically effective amount of a platinum-containing catalyst. The compositions will be essentially free of fillers. The most preferred embodiments of component (c) will be those wherein (i) is of the formula:

$$(R^6)_a(R^7)_b\ SiO_{\frac{4-a-b}{2}} \quad ; \text{ and}$$

(ii) is of the formula

$$(R^6)_a(H)_b\ SiO_{\frac{4-a-b}{2}}$$

where $R^6$ is selected from monovalent hydrocarbon radicals free of olefinic unsaturation, halogenated monovalent hydrocarbon radicals free of olefinic unsaturation and cyanoalkyl radicals, e.g., of from 1 to 30, preferably from 1 to 6 carbon atoms; $R^7$ is selected from vinyl and allyl radicals; a has a value of from 1.0 to 2.5; b has a value of from 0.005 to 2.0; and the sum of a plus b is equal to from 1.005 to 3.0. In the most preferred embodiments, (iii) will be selected from chloroplatinic acid, platinum black, platinum adsorbed on a carrier, a platinum-olefin complex, a platinum-cyclopropane complex or a mixture of any of the foregoing.

In another aspect of the present invention there are provided thermocurable radiopaque compositions comprising a Part A comprised of:

(a) a resin which includes from 100 to 0 parts by weight of a thermoplatic polyurethane and, correspondingly, from 0 to 100 parts of a polymer of a halogenated vinyl monomer per 100 parts by weight of resin;

(b) a radiopacifier therefor consisting of a diiodobenzoate or a tetraiodobenzoate compound of formula (I) or a triiodobenzoate of formula (II):

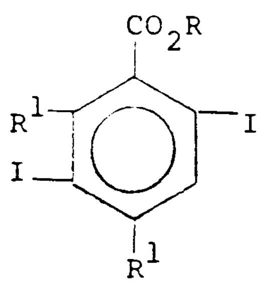

(I)

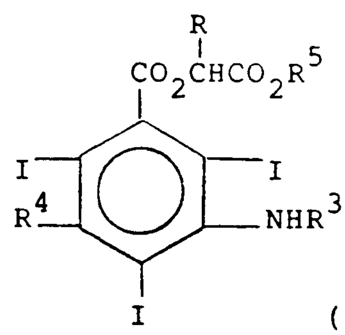

(II)

wherein R is alkyl or alkoxy-alkyl, $R^1$ is hydrogen or iodo, $R^2$ is hydrogen or alkyl, $R^3$ is hydrogen or alkanoyl, $R^4$ is hydrogen or alkanoylamino, and $R^5$ is alkyl, or a mixture of said compounds, said radiopacifier comprising 10 to 40 parts by weight per 100 parts by weight of (a) and (b); and

(c) (i) an organic silicon compound having at least two silicon-bonded unsaturated groups per molecule; and a Part B comprised of:

(a) a resin which includes from 100 to 0 parts by weight of a thermoplatic polyurethane and, correspondingly, from 0 to 100 parts of a polymer of a halogenated vinyl monomer per 100 parts by weight of resin;

(b) a radiopacifier therefor consisting of a diiodobenzoate or a tetraiodobenzoate compound of formula (I) or a triiodobenzoate of formula (II):

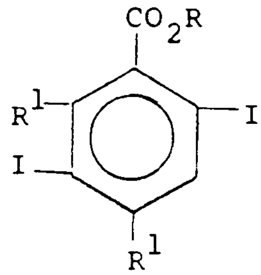

(I)

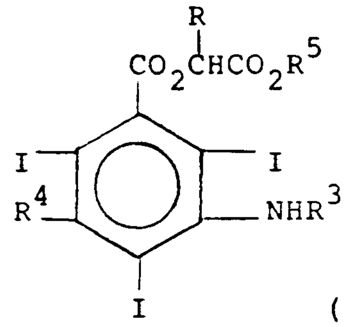

(II)

wherein R is alkyl or alkoxy-alkyl, $R^1$ is hydrogen or iodo, $R^2$ is hydrogen or alkyl, $R^3$ is hydrogen or alkanoyl, $R^4$ is hydrogen or alkanoylamino, and $R^5$ is alkyl, or a mixture of said compounds, said radiopacifier comprising 10 to 40 parts by weight per 100 parts by weight of (a) and (b); and

(c) (ii) a silicon compound containing at least two silicon-bonded hydrogen atoms per molecule; and

(c)(iii) a small, catalytically effective amount of a platinum-containing catalyst, wherein the respective amounts of (c)(i), (ii) and (iii) are selected to provide from about 8 to about 30 parts by weight of a platinum-cured silicone network polymer, after having blended and thermoformed parts A and B, per 100 parts by weight of the total composition. The preferred embodiments of the above thermocurable radiopaque compositions will be those wherein component (b) in part A and/or part B has as each $R^1$ an iodo and as R a (lower) alkyl or (lower)alkyl(lower)alkoxy. The most preferred embodiments will have (b), (c) (i) (ii) (iii) as previously described.

The thermoplastic polyurethanes are known to those skilled in this art. They are described in the Encyclopedia of Polymer Science and Technology, Vol. 11, pages 506-563, New York, Interscience Publishers, 1969, and especially on page 549. They are also commercially available from a number of suppliers, such as Hooker, as Rucothenes®, especially 3713, and from Upjohn, as Pellethene®, e.g., 75D, 85D and X-010155 80D, and from Mobay, as Texin®.

Suitable vinyl resins are described in the above-mentioned U.S. Patent No. 3,645,955. They are available from a number of sources, such as Escambia®, Nos. 3225,3250 and 3255; Diamond No. 450 and No. 500; Borden, 106PM and Dow Chemical Co. 100-4.

The di- and tetra-iodobenzoate radiopacifier compounds (b) (I) can be made by procedures fully described in the above mentioned U.S. Patent Nos. 3,645,955 and 3,361,700. In general, a 2,3,4,6-tetraiodobenzoyl halide or 2,5-diiodobenzoyl halide will be treated with an alkanol or alkoxyalkanol at ordinary temperatures, preferably in the presence of an acid acceptor. Alternatively, the free acid can be reacted with a sodium alkoxide or alkoxyalkoxide. The product can be recovered and purified in a known way, e.g., distillation.

The triiodobenzoate radiopacifier compounds (b) (II) can be made by procedures fully described in the above-mentioned U.K. Patent No. 866,184. In general a known acid of the formula

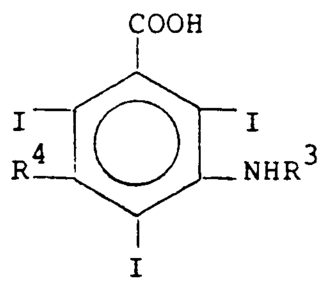

wherein $R^2$ and $R^3$ are defined above, in the form of a metal or ammonium salt is caused to react with an alkyl alphahaloalkanoate of the formula $XCH(R^2)COOR^5$ wherein X is halogen, e.g., chlorine, bromine or iodine and $R^2$ and $R^5$ are as defined above, to produce the compound of formula 2. As is described in the U.K. Patent No. 866,184, Example 20, the compound of formula II wherein $R^2$ is H, $R^3$ is H, $R^4$ is H and $R^5$ is $C_2H_5$, namely, ethyl(3-amino-2,4,6-triiodobenzoyloxy)acetate, can be made by reacting 3-amino-2,4,6-triiodobenzoic acid with ethylchloroacetate. As is described in Example 23 of the U.K. Patent No. 866,184, the compound of formula II wherein $R^2$ is $C_2H_5$, $R^3$ is H, $R^4$ is H and $R^5$ is $C_2H_5$, namely, ethyl 2-(3-amino-2,4,6-triiodobenzoyloxy)butanoate, can be made by reacting 3-amino-2,4,6-triiodobenzoic acid with ethyl-alpha-bromo-n-butyrate.

The platinum-cured silicone network polymers employed herein are known in this art as components of interpenetrating polymer networks (IPN's). The compositions are blends of crosslinked polymers containing essentially no covalent bonds, or grafts, between them. In the present instance the IPN's are made by combining the thermoplastic resin, e.g., polyurethane, alone, or vinyl resin, alone, or in combinations thereof, with crosslinkable silicon compounds. As will be shown hereinafter, by specific examples and by reference to patents, platinum-curable two-part silicone polymers are well known in this art. The formation of IPN-modified silicone/resin thermoplastics is discussed in Arkles, "IPN-Modified Silicone Thermoplastics" MD & DI, November, 1983, pages 66-70; Arkles and Carreno, "Silicone IPN & Modified Thermoplastics", ANTEC 84 Conference Proceedings, New Orleans April 30-May 3, 1984, pages 486-487; and Arkles, Ibid., pages 1080-1082, and Arkles, U.S. Patent No. 4,500,688.

Suitable silicon compounds containing at least two silicon-bonded hydrogen atoms per molecule which can be employed in the practice of the invention have the formula:

$$(R^6)_a(H)_b\,SiO_{\frac{4-a-b}{2}}$$

where $R^6$ is an organic radical attached to silicon through silicon-carbon linkages and free of aliphatic unsaturation. Among the organic radicals represented by $R^6$ are monovalent hydrocarbon radicals free of olefinic unsaturation, halogenated monovalent hydrocarbon radicals free of olefinic unsaturation and cyanoalkyl radicals. More particularly, radicals represented by R include alkyl radicals, e.g., methyl, ethyl, propyl, butyl, octyl, etc. radicals; cycloalkyl radicals, e.g., cyclohexyl, cycloheptyl, etc. radicals; aryl radicals, e.g., phenyl, naphthyl, tolyl, xylyl, etc. radicals; aralkyl radicals, e.g., benzyl, phenylethyl, phenylpropyl, etc. radicals; halogenated radicals of the above types, including chloromethyl, chloropropyl, chlorophenyl, dibromophenyl, etc. radicals; and cyanoalkyl radicals, e.g., betacyanoethyl, gamma-cyanopropyl, betacyanopropyl, etc. radicals. Preferably, at least 50 percent of the $R^6$ groups attached to silicon in the polysiloxane of the above formula are methyl radicals, with up to 50 percent of the $R^6$ radicals being phenyl radicals.

The silicon compounds containing at least two silicon-bonded hydrogen atoms per molecule are well known in the art and include such materials as 1,3-dimethyldisiloxane, 1,1,3-trimethyldisiloxane, 1,1,3,3-tetramethyldisiloxane as well as higher polymers containing up to 100,000 or more silicon atoms per molecule. Also included within the scope of the above formula are cyclic materials such as the cyclic trimer or cyclic tetramer of methyl hydrogen siloxane. Such silicon compounds containing at least two silicon-bonded hydrogen atoms per molecule are described in Ashby, U.S. Patent No. 3,159,601.

Suitable organic silicon compounds containing at least two silicon-bonded vinyl or allyl groups per molecule which can be employed in the practice of the present invention are also well known in the art and are characterized by the formula:

$$(R^6)_a(R^7)_b\,SiO_{\frac{4-a-b}{2}}$$

where $R^6$ is as previously defined and $R^7$ is a member selected from vinyl and allyl radicals.

The preparation of these vinyl- or allyl-containing silicone compounds within the scope of the above formula is well known in the art. Included within the scope of the siloxanes of the above formula are low molecular weight materials such as 1,1-divinyltetramethyldisiloxane, 1,3-divinyltetramethyldisiloxane, 1,1,3-trivinyltrimethylsiloxane, 1,1,3,3-tetravinyldimethyldisiloxane as well as higher polymers containing up to 100,000 or more silicon atoms per molecule. Also included within the scope of the unsaturated organopolysiloxanes within the scope of the above formula are cyclic materials containing silicon-bonded vinyl or allyl radicals, such as the trimer, tetramer or pentamer, of methylvinylsiloxane or methylallylsiloxane. Such organic silicon compounds containing at least two silicon bonded vinyl or allyl groups per molecule are described in Ashby, U.S. Patent No. 3,159,601.

Any number of platinum-containing materials will catalyze the addition of silicon-bonded hydrogen

atoms across the double bonds of compounds containing olefinic unsaturation. For example, Bailey, U.S. Patent 2,970,150, shows the use of a platinum-on-charcoal catalyst, Speier, et al., U.S. Patent 2,823,218, show the use of chloroplatinic acid, and Ashby, U.S. Patent 3,159,622, shows the use of a platinum-cyclopropane complex. These and many other platinum-containing catalysts may be employed in the practice of the present invention. Preferred platinum-containing catalysts will be those described in Ashby, U.S. 3,159,601, which describes platinum-olefin complexes having the formulae:

(1) $[PtCl_2 \bullet \text{Olefin}]_2$
(2) $H[PtCl_3 \bullet \text{Olefin}]$

wherein the olefin portion of (1) and (2) may be any olefin, but is preferably an alkene having from 2 to 8 carbon atoms, a cycloalkene having from 5 to 7 carbon atoms, or styrene.

When used in the appended claims, the term "alkyl" contemplates straight and branched chain radicals of from 1 to 30 carbon atoms, and the term "(lower)alkyl" contemplates such groups of from 1 to 6 carbon atoms.

The term "radioparent" plasticizer means a conventional plasticizer, for example, a dialkyl ester of aromatic or aliphatic polybasic acids, e.g., dioctyl adipate, which is commercially available from Rohm & Haas Co., as Monoplex® DIOA. Also contemplated are epoxy plasticizers, such as Swift & Co.'s Epoxol® 9-5. The term "heat stabilizer for said vinyl resin" embraces metallic salts, based on tin, calcium, zinc and the like. A suitable commercial heat stabilizer is based on calcium and zinc, available from Advance Division of Carlisle Chemical Works, as CZ-11C.

The formulations will be aided in some cases by the inclusion of lubricants such as metallic stearate, stearic acid, paraffin wax, mineral oil, etc., in conventional amounts. See U.S. Patent No. 3,645,955, incorporated herein to minimize unnecessarily detailed description.

The compositions are prepared and converted into useful products by techniques well known to those skilled in the art.

In one manner of proceeding, the fluid ingredients, e.g., radiopaque compound(s) if liquid, are blended with the powdered solids, e.g., thermoplastic polyurethane resin, reactive curable silicone components, and/or vinyl resin, stabilizers and plasticizers and then fused and mixed under pressure, e.g., in a Banbury-type mixer and discharged. Conventional 2-roll compounding techniques can also be used. The composition is cooled and granulated.

If extrusions are to be made, the granulated composition can be fed to a conventional machine, for example, a 30 millimeter Reifenhouser-type single screw extruder operated at suitable temperature, e.g., 137,78-166,56 °C (280 °-330 °F.) and the tubing or other shapes formed at a suitable rate, e.g., 2133,6-3048 meters (7,000-10,000 feet) per hour and cut automatically to length.

In another manner of proceeding, a thermocurable radiopaque composition comprising a part A and a part B, previously described, may be prepared and extruded, with or without precompounding either A and B, as mentioned above. Optionally, A and B will be precompounded and thereafter extruded under suitable conditions.

In still another manner of proceeding, multiwall tubing, of uniform or co-tapered wall construction, may be prepared by co-extruding a thermocurable radiopaque composition, comprising a part A and a part B, with another thermo plastic resin, or with another, differently formulated, thermocurable radiopaque composition comprising a part A and a part B.

Those skilled in the art will prepare a variety of thermocurable compositions comprising A and B wherein components (c)(i), (ii) and (iii), previously described, may be varied to effect different cure rates, degrees of crosslinking and/or physical/chemical properties of the finished compositions.

As is pointed out above, the compositions of this invention can be used for many various and diverse purposes, e.g., protective sheeting, surgeon's gloves, intubation sets, heart catheters, stomach tubes, nasal tubes, thoracic catheters and the like. The following examples primarily illustrate the use of these compositions in the form of single and multiple wall surgical tubing. However, from the foregoing description and the following examples and by reference to other well known teachings, the methods and modes by which the plasticized radiopaque resin compositions of this invention can be formed into various other articles will be readily apparent to those skilled in the art.

The medical grade radiopaque tubing prepared as described in the following examples is non-toxic, non-reactive to tissue and may be sterilized by gas, cold sterilization solutions or heat sterilization techniques. The tubing is generally dispensed as such and the surgeon or trained technician will form it into catheters for roentgenography. For maximum convenience, the tubing can also be preformed into articles and dispensed, e.g., as sterile disposable intravenous catheters.

By way of illustration, catheters according to this invention will be fabricated from the medical-surgical tubing of the following examples by operations comprising tip forming, tip finishing, shaping, side hole forming, and flaring. Before use they will be sterilized.

Those skilled in the art will prepare a variety of tip shapes. For internal mammary and axillary artery branches a three-quarter loop is formed in the distal end. For precutaneous arteriography and cerebral arteriography via femoral, a 45°-60° smooth bend will be formed in the distal end. Selective renal arteriography and celiac arteriography requires a one-half loop. Hepatic venography uses about a seven-eighths loop. For trans-sepal left-heart catheterization via the femoral vein, a three-quarter loop, like that above-described for mammary branches, but larger, is formed. On the other hand, abdominal aortography via brachial artery uses a rather large, one-third closed loop and thoracic aortogrphy via the femoral artery uses the same shape but bigger. For lumbar aortography via the femoral artery the tip is straight out. For coronary arteriography, the end of the catheter is looped.

The heavier-walled tubing is formed into such typical shapes by inserting a forming wire within the tubing and heating in a tiny flame until visibly softened. By pulling from both ends the tubing is drawn to the wire and forms a uniform lumen around it. The tip is formed by cutting, e.g., with a razor blade, at the drawn diameter and is smoothly rounded in the flame. Next a precurved wire is inserted into the tube which is then immersed in hot water until the tubing softens. Quenching in cold water will cause the catheter to conform to the curve of the forming wire. Side hole or eye punching is accomplished by rotating a sharpened hole punch cannula under slight pressure. The holes should be large enough to expel contrast media without excess build up of injection pressures but should be no larger than 2/3 of the internal diameter of the tubing. The catheter is cut to the preselected length and flared. Heating in a small flame and rotating on a flaring tool produces a flare of the desired size. The catheter can be bonded at the flare, e.g., with epoxy cement, to a suitable hub. On the other hand, an adapter can be used to screw the catheter to a Luer-Lok stopcock, or the like.

## DESCRIPTION OF THE PREFERRED EMBODIMENTS

The following non-limiting examples illustrate the present invention.

### EXAMPLE 1

| PART A | 1 |
|---|---|
| Silicone-network polymer | (12%) |
| Composition (parts by weight) | |
| Resin, thermoplastic polyurethane, Pellethane 75 durometer | 1800 |
| 2-Ethoxyethyl-2,5-diiodobenzoate | 500 |
| Methylvinyl polysiloxane[a] | 315 |

| | |
|---|---|
| PART B | |
| Silicone-network polymer | (12%) |
| Composition (parts by weight) | |
| Resin, thermoplastic polyurethane, Pellethane 75 durometer | 1800 |
| 2-Ethoxyethyl-2,5-diiodobenzoate | 500 |
| Methylhydrogen polysiloxane[b] | 315 |
| Platinum-olefin complex catalyst[c] | .031 |

a e.g., Methylvinylpolysiloxane fluid, 0,2 $Ns/m^2$ (200 cps) @25°C comprising trimethylsilyl chain-stopped and consisting of 2.0 mole percent methylvinylsiloxane units with the remainder of the diorganosiloxane units being dimethylsiloxane units. Example 8, U.S. Pat. 3,159,601.

b e.g., Methylhydrogenpolysiloxane fluid, 0,2 $Ns/m^2$ (200 cps) @25°C., comprising trimethylsilyl chain-stopped and consisting of 2.0 mole percent methylhydrogensiloxane units with the remainder of diorganosiloxane units being dimethylsiloxane units. Example 8, U.S. Pat. 3,159,601.

c $(Pt\,Cl_2 \cdot C_2H_4)_2$ Example 2, U.S. 3,159,601.

The compositions are mixed in a Banbury mixer for 4 minutes at 173,89 °C (345 °F) and for 2 minutes at 154,45 °C 310 °F., discharges, and granulated through a 3,2 mm (1/8 in.) screen.

The granulated products of Parts A and B are dry blended at a 1:1 ratio and are thereafter extruded in a 30 mm. Reifenhauser screw extruder at 154,45 °C-165,56 °C (310 °-330 °F.) into medical-surgical single wall tubing of the type shown in FIG. 2, 1,65 mm O.D x 1,17 mm I.D. - 0,25 mm wall thickness (0.065 O.D. x 0.044 I.D. - 0.0105"), having properties eminently suitable for use as intubation sets and in catheter needles.

EXAMPLES 2-4

The general procedure of Example 1 is repeated, substituting the formulations enumerated in Table 1, and soft tubings according to this invention are obtained:

Table 1: Radiopaque Soft Urethane/Silicone Compositions

| Example | 2 | 3 | 4 |
|---|---|---|---|
| Silicone-network polymer | (10%) | (25%) | (18%) |
| PART A | | | |
| Composition (parts by weight) | | | |
| Resin, thermoplastic polyurethene, | | | |
| Pellethane 75 D[a] | 1800 | 1200 | 1800 |
| X-010155 80D[b] | -- | 600 | -- |
| 2-Ethoxyethyl 2,5-diiodobenzoate | 900 | 1200 | 1038 |
| Methylvinyl polysiloxane[c] | 300 | 1000 | 632 |
| PART B | | | |
| Composition (parts by weight) | | | |
| Resin, thermoplastic polyurethane | | | |
| Pellethane 75 D[a] | 1800 | 1200 | 1800 |
| X-010155 80D[b] | -- | 600 | -- |
| 2-Ethoxyethyl 2,5-diiodobenzoate | 900 | 1200 | 1038 |
| Methylhydrogen polysiloxane[d] | 300 | 1000 | 632 |
| Platinum-olefin complex catalyst[e] | .030 | 0.100 | .063 |

a Upjohn Co.

b Upjohn Co.

c-e See Example 1, footnote a-c

EXAMPLES 5-7

The general procedure of Example 1 is repeated, substituting the formulations enumerated in Table 2, and medium hard tubings according to this invention are obtained:

Table 2: Radiopaque Medium Hard Urethane/Silicone Compositions

| Example | 5 | 6 | 7 |
|---|---|---|---|
| Silicone-network polymer | (20%) | (15%) | (25%) |
| PART A | | | |
| Composition (parts by weight) | | | |
| Resin, thermoplastic polyurethane, | | | |
| Pellethane 75 D[a] | 900 | 982 | 600 |
| X-010155 80D[b] | 900 | 818 | 1200 |
| 2-Ethoxyethyl 2,5-diiodobenzoate | 1080 | 982 | 600 |
| n-Butyl 2,3,4,6-tetraiodobenzoate | -- | -- | 600 |
| Methylvinyl polysiloxane[c] | 720 | 492 | 1000 |
| PART B | | | |
| Composition (parts by weight) | | | |
| Resin, thermoplastic polyurethane | | | |
| Pellethane 75 D[a] | 900 | 982 | 600 |
| X-010155 80D[b] | 900 | 818 | 1200 |
| 2-Ethoxyethyl 2,5-diiodobenzoate | 1080 | 982 | 600 |
| n-Butyl 2,3,4,6-tetraiodobenzoate | -- | -- | 600 |
| Methylhydrogen polysiloxane[d] | 720 | 492 | 1000 |
| Platinum-olefin complex catalyst[e] | 0.072 | 0.049 | 0.100 |

a Upjohn Co.
b Upjohn Co.
c-e See Example 1, footnote a-c

EXAMPLES 8-10

The general procedure of Example 1 is repeated, substituting the formulations enumerated in Table 3, and hard tubings according to this invention are obtained:

Table 3: Radiopaque Hard Urethane/Silicone Compositions

| Example | 8 | 9 | 10 |
|---|---|---|---|
| Silicone-network polymer | (25%) | (18%) | (10%) |
| PART A | | | |
| Composition (parts by weight) | | | |
| Resin, thermoplastic polyurethane, | | | |
| Pellethane 75 D[a] | -- | 519 | 600 |
| X-010155 80D[b] | 1800 | 1281 | 1200 |
| 2-Ethoxyethyl 2,5-diiodobenzoate | 900 | 519 | 300 |
| n-Butyl 2,3,4,6-tetraiodobenzoate | -- | 519 | 600 |
| Methylvinyl polysiloxane[c] | 900 | 624 | 300 |
| PART B | | | |
| Composition (parts by weight) | | | |
| Resin, thermoplastic polyurethane | | | |
| Pellethane 75 D[a] | -- | 519 | 600 |
| X-010155 80D[b] | 1800 | 1281 | 1200 |
| 2-Ethoxyethyl 2,5-diiodobenzoate | 900 | 519 | 300 |
| n-Butyl 2,3,4,6-tetraiodobenzoate | -- | 519 | 600 |
| Methylhydrogen polysiloxane[d] | 900 | 624 | 300 |
| Platinum-olefin complex catalyst[e] | 0.090 | 0.062 | 0.030 |

a Upjohn Co.
b Upjohn Co.
c-e See Example 1, footnote a-c

EXAMPLES 11-12

The general procedure of Example 1 is repeated substituting in the formulation for the 2-ethoxyethyl 2,4-diiodobenzoate, equal weights, respectively, of ethyl (3-amino-2,4,6-triiodobenzoyloxy)acetate, and ethyl 2-(3-amino-2,4,6-triiodobenzoyloxy)butanoate, and tubings according to this invention are obtained.

EXAMPLES 13-14

The general procedure of Example 1 is repeated, substituting the formulations enumerated in Table 4, and polyvinyl chloride/polyurethane tubings according to this invention are obtained.

Table 4: Radiopaque Vinyl Chloride/ Urethane/Silicone Compositions

| Example | 13 | 14 |
|---|---|---|
| Silicone-network polymer | (10%) | (20%) |
| **PART A** | | |
| Composition (parts by weight) | | |
| Resin, thermoplastic polyurethane, | | |
| Pellethane 75 D[a] | 964 | 783 |
| X-010155 80D[b] | -- | 391 |
| Resin, thermoplastic polyvinylchloride, BK 80[c] | 836 | 626 |
| 2-Ethoxyethyl 2,5-diiodobenzoate | 482 | 626 |
| n-Butyl 2,3,4,6-tetraiodobenzoate | 482 | 1174 |
| Heat stabilizer, CZ-11C[d] | 16 | 16 |
| Plasticizer, Epoxol 905[e] | 112 | 141 |
| Methylvinyl polysiloxane[f] | 321 | 783 |
| **PART B** | | |
| Composition (parts by weight) | | |
| Resin, thermoplastic polyurethane | | |
| Pellethane 75 D[a] | 964 | 783 |
| X-010155 80D[b] | -- | 391 |
| Resin, thermoplastic polyvinylchloride, BK 80[c] | 836 | 626 |
| 2-Ethoxyethyl 2,5-diiodobenzoate | 482 | 1174 |
| n-Butyl 2,3,4,6-tetraiodobenzoate | 482 | -- |
| Heat stabilizer, CZ-11C[d] | 16 | 16 |
| Plasticizer, Epoxol 905[e] | 112 | 141 |
| Methylhydrogen polysiloxane[g] | 321 | 783 |
| Platinum-olefin complex catalyst[h] | 0.030 | 0.078 |

a-b Upjohn Co.

c Goodyear Co.

d Advance Division of Carlisle Chemical Works

e Swift & Co.

f-h See Example 1, footnote a-c

EXAMPLE 15 (Silicone network polymer - 20%)

PART A

Composition (parts by weight)

| | |
|---|---|
| Resin, thermoplastic polyvinylchloride, BK 80[a] | 1800 |
| 2-Ethoxyethyl-2,5-diiodobenzoate | 600 |
| n-Butyl 2,3,4,6-tetraiodobenzoate | 600 |
| Plasticizer, Epoxol® 9-5[b] | 160 |
| Heat stabilizer, CZ-11C[c] | 40 |
| Methylvinyl polysiloxane[d] | 80 |

PART B

Composition (parts by weight)

| | |
|---|---|
| Resin, thermoplastic polyvinylchloride, BK 80[a] | 1800 |
| 2-Ethoxyethyl-2,5-diiodobenzoate | 600 |
| n-Butyl 2,3,4,6-tetraiodobenzoate | 600 |
| Plasticizer, Epoxol 9-5[b] | 160 |
| Heat stabilizer, CZ-11C[c] | 40 |
| Methylhydrogen polysiloxane[e] | 80 |
| Platinum-olefin complex catalyst[f] | 0.080 |

a Goodyear Corp.

b Advance Division of Carlisle Chemical Works.

c Swift & Co.

d e.g., Methylvinylpolysiloxane fluid, 200 cps @25°C. comprising trimethylsilyl chain-stopped and consisting of 2.0 mole percent methylvinylsiloane units with the remainder of the diorganosiloxane units being dimethylsiloxane units. Example 8, U.S. Pat. 3,159,601.

e e.g., Methylhydrogenpolysiloxane fluid, 200 cps @25°C., comprising trimethylsilyl chain-stopped and consisting of 2.0 mole percent methylhydrogen siloxane units with the remainder of the diorganosiloxane units being dimethylsiloxane units. Example 8, U.S. Patent 3,159,601.

f $(Pt\ Cl_2 \cdot C_2H_4)_2$ Example 2, U.S. 3,159,601.

The fluids are mixed in a planetary mixer, e.g., a Hobart mixer, for 15 minutes. The powdered ingredients are added and mixed for 1.5 hours. The mixture is fluxed and mixed in a Banbury - one 10-15 min. cycle - and discharged. The partially cooled mass is granulated through 3,2 mm (1/8 in.) or 6,4 mm (1/4 in.) screen. The granulated product is extruded in a 30 mm. Reifenhauser single screw extruder at 2133,6-3048 meters (7,000-10,000 feet) per hour into medical-surgical tubing, 1,65 mm O.D. x 1,17 mm I.D.

- 0,25 mm wall thickness (0.065 O.D. x 0.046 I.D. - wall 0.010) exceeding minimum specifications of tensile break strength, elongation and flexural strength for use as intubation sets and in catheter needles.

EXAMPLE 16

The general procedure of Example 15 is repeated, substituting the formulation below, and a medium hard tubing according to this invention is obtained.

PART A

(Silicone network polymer - 10%)

| Composition (parts by weight) | |
|---|---|
| Resin, thermoplastic polyvinylchloride, BK 80[a] | 1800 |
| 2-Ethoxyethyl-2,5-diiodobenzoate | 491 |
| n-Butyl 2,3,4,6-tetraiodobenzoate | 491 |
| Plasticizer, Epoxol 9-5[b] | 131 |
| Heat stabilizer, CZ-11C[c] | 33 |
| Methylvinyl polysiloxane[d] | 327 |

PART B

| Composition (parts by weight) | |
|---|---|
| Resin, thermoplastic polyvinylchloride, BK 80 | 1800 |
| 2-Ethoxyethyl-2,5-diiodobenzoate | 491 |
| n-Butyl 2,3,4,6-tetraiodobenzoate | 491 |
| Plasticizer, Epoxol 9-5[b] | 131 |
| Heat stabilizer, CZ-11C[c] | 33 |
| Methylhydrogen polysiloxane[e] | 327 |
| Platinum-olefin complex catalyst[f] | 0.03 |

a-f See Example 15, footnotes a-f.

EXAMPLE 17

The general procedure of Example 15 is repeated, substituting the formulation below, and a soft tubing according to this invention is obtained.

PART A

(Silicone network polymer - 20%)

| Composition (parts by weight) | |
|---|---|
| Resin, thermoplastic polyvinylchloride, BK 80[a] | 1800 |
| 2-Ethoxyethyl-2,5-diiodobenzoate | 1200 |
| n-Butyl 2,3,4,6-tetraiodobenzoate | -- |
| Plasticizer, Epoxol 9-5[b] | 160 |
| Heat stabilizer, CZ-11C[c] | 40 |
| Methylvinyl polysiloxane[d] | 800 |

PART B

| Composition (parts by weight) | |
|---|---|
| Resin, thermoplastic polyvinylchloride, BK 80 | 1800 |
| 2-Ethoxyethyl-2,5-diiodobenzoate | 1200 |
| n-Butyl 2,3,4,6-tetraiodobenzoate | -- |
| Plasticizer, Epoxol 9-5[b] | 160 |
| Heat stabilizer, CZ-11C[c] | 40 |
| Methylhydrogen polysiloxane[e] | 800 |
| Platinum-olefin complex catalyst[f] | 0.08 |

a-f See Example 15, footnotes a-f.

EXAMPLE 18

The general procedure of Example 15 is repeated, substituting the fomulation below, and a hard tubing according to this invention is obtained.

PART A

(Silicone network polymer - 5%)

| Composition (parts by weight) | |
|---|---|
| Resin, thermoplastic polyvinylchloride, BK 80[a] | 1800 |
| 2-Ethoxyethyl-2,5-diiodobenzoate | 600 |
| n-Butyl 2,3,4,6-tetraiodobenzoate | 300 |

| | |
|---|---|
| Plasticizer, Epoxol 9-5[b] | 120 |
| Heat stabilizer, CZ-11C[c] | 30 |
| Methylvinyl polysiloxane[d] | 150 |

PART B

Composition (parts by weight)

| | |
|---|---|
| Resin, thermoplastic polyvinylchloride, BK 80 | 1800 |
| 2-Ethoxyethyl-2,5-diiodobenzoate | 600 |
| n-Butyl 2,3,4,6-tetraiodobenzoate | 300 |
| Plasticizer, Epoxol 9-5[b] | 120 |
| Heat stabilizer, CZ-11C[c] | 30 |
| Methylhydrogen polysiloxane[e] | 150 |
| Platinum-olefin complex catalyst[f] | 0.015 |

a-f See Example 15, footnotes a-f.

EXAMPLE 19

The general procedure of Example 15 is repeated, substituting the formulation below, and a medium hard tubing according to this invention is obtained.

PART A

(Silicone network polymer - 12%)

Composition (parts by weight)

| | |
|---|---|
| Resin, thermoplastic polyvinylchloride, No. 3225[a] | 1800 |
| 2-Ethoxyethyl-2,5-diiodobenzoate | -- |
| n-Butyl 2,3,4,6-tetraiodobenzoate | 1275 |
| Plasticizer, Epoxol 9-5[b] | 188 |
| Heat stabilizer, CZ-11C[c] | 38 |
| Methylvinyl polysiloxane[d] | 450 |

PART B

Composition (parts by weight)

| | |
|---|---|
| Resin, thermoplastic polyvinylchloride, No. 3225 | 1800 |
| 2-Ethoxyethyl-2,5-diiodobenzoate | -- |
| n-Butyl 2,3,4,6-tetraiodobenzoate | 1275 |
| Plasticizer, Epoxol 9-5[b] | 188 |
| Heat stabilizer, CZ-11C[c] | 38 |

| | |
|---|---|
| Methylhydrogen polysiloxane[e] | 450 |
| Platinum-olefin complex catalyst[f] | 0.045 |

a Escambia Corp.

b-f See Example 15, footnotes b-f.

EXAMPLE 20

A multi-wall radiopaque vinyl medical-surgical tubing of 2,79 mm (0.110 inch) outer diameter and 1,80 mm (0.071 inch) inner diameter is extruded in a conventional manner employing a bi-orifice tubular die for co-extrusion of two concentric bonded tubings wherein the inner tube has a thickness of 4,45 mm (0.0175 inch) and the outer shell has a thickness of 0,05 mm (0.002 inch).

Inner Core (Silicone network polymer - 18%)

PART A

| Composition (parts by weight) | |
|---|---|
| Resin, thermoplastic polyvinylchloride, 100-4[a] | 1800 |
| 2-Ethoxyethyl-2,5-diiodobenzoate | -- |
| n-Butyl 2,3,4,6-tetraiodobenzoate | 1350 |
| Radioparent plasticizer, Monoplex DIOA[b] | 360 |
| Plasticizer, Epoxol 9-5[c] | 144 |
| Heat stabilizer, CZ-11C[d] | 36 |
| Methylvinyl polysiloxane[e] | 810 |

PART B

| Composition (parts by weight) | |
|---|---|
| Resin, thermoplastic polyvinylchloride, 100-4 | 1800 |
| 2-Ethoxyethyl-2,5-diiodobenzoate | -- |
| n-Butyl 2,3,4,6-tetraiodobenzoate | 1350 |
| Radioparent plasticizer, Monoplex DIOA | 360 |
| Plasticizer, Epoxol 9-5[b] | 144 |
| Heat stabilizer, CZ-11C[c] | 36 |
| Methylhydrogen polysiloxane[f] | 810 |
| Platinum-olefin complex catalyst[g] | 0.081 |

Outer Shell (Silicone network polymer - 12.5%)

PART A

| Composition (parts by weight) | |
|---|---|
| Resin, thermoplastic polyvinylchloride, 100-4[a] | 1800 |
| 2-Ethoxyethyl-2,5-diiodobenzoate | -- |
| n-Butyl 2,3,4,6-tetraiodobenzoate | 2520 |
| Radioparent plasticizer, Monoplex DIOA[b] | -- |
| Plasticizer, Epoxol 9-5[c] | 144 |
| Heat stabilizer, CZ-11C[d] | 36 |
| Methylvinyl polysiloxane[e] | 643 |

PART B

| Composition (parts by weight) | |
|---|---|
| Resin, thermoplastic polyvinylchloride, 100-4 | 1800 |
| 2-Ethoxyethyl-2,5-diiodobenzoate | -- |
| n-Butyl 2,3,4,6-tetraiodobenzoate | 2520 |
| Radioparent plasticizer, Monoplex DIOA | -- |
| Plasticizer, Epoxol 9-5[b] | 144 |
| Heat stabilizer, CZ-11C[c] | 36 |
| Methylhydrogen polysiloxane[f] | 643 |
| Platinum-olefin complex catalyst[g] | 0.064 |

a Dow Chemical Co.

b Rohm and Haas Co.

c-g See Example 15, footnotes b-f.

This vinyl multi-wall medical-surgical tubing is found to have the described desired properties necessary for medical-surgical tubing.

EXAMPLE 21

A multi-wall radiopaque vinyl medica-surgical tubing particularly useful for thoracic, stomach and nasal use having an outer diameter of 8,84 mm (0.348 inch) and an inner diameter of 6,4 mm (0.25 inch) is extruded in a conventional manner employing a bi-orifice tubular die for co-extrusion of two concentric bonded tubings wherein the inner tube has a thickness of 0,99 mm (0.039 inch) and the outer jacket has a thickness of 0,216mm (0.0085 inch).

Inner Core (Silicone network polymer - 10%)

PART A

| Composition (parts by weight) | |
|---|---|
| Resin, thermoplastic polyvinylchloride, 100-4[a] | 1800 |
| Radioparent plasticizer, dioctyl phthalate | 1188 |
| Plasticizer, Epoxol 9-5[b] | 540 |
| Heat stabilizer, CZ-11C[c] | 72 |
| Methylvinyl polysiloxane[d] | 400 |

PART B

| Composition (parts by weight) | |
|---|---|
| Resin, thermoplastic polyvinylchloride, 100-4 | 1800 |
| Radioparent plasticizer, dioctyl phthalate | 1188 |
| Plasticizer, Epoxol 9-5 | 540 |
| Heat stabilizer, CZ-11C[c] | 72 |
| Methylhydrogen polysiloxane[f] | 400 |
| Platinum-olefin complex catalyst[g] | 0.040 |

Outer Shell (Silicone network polymer - 10%)

PART A

| Compositión (parts by weight) | |
|---|---|
| Resin, thermoplastic polyvinylchloride, 100-4[a] | 1800 |
| 2-Ethoxyethyl-2,5-diiodobenzoate | 1260 |
| Plasticizer, Epoxol 9-5[b] | 162 |
| Heat stabilizer, CZ-11C[c] | 32 |
| Methylvinyl polysiloxane[d] | 360 |

PART B

| Composition (parts by weight) | |
|---|---|
| Resin, thermoplastic polyvinylchloride, 100-4 | 1800 |
| 2-Ethoxyethyl-2,5-diiodobenzoate | 1260 |
| Plasticizer, Epoxol 9-5 | 162 |
| Heat stabilizer, CZ-11C | 32 |
| Methylhydrogen polysiloxane[e] | 360 |
| Platinum-olefin complex catalyst[f] | 0.036 |

a Dow Chemical Co.

b-f See Example 15, footnotes b-f.

This multi-wall medical-surgical tubing has a slick, glass-like surface. It is pale rose tan and clear. Radiopacity is excellent. The composition and tubing are useful in this size for thoracic catheters.

EXAMPLE 22

A multi-wall radiopaque vinyl medical-surgical tubing according to this invention is extruded in a conventional manner employing a bi-orifice tubular die for co-extrusion of two concentric bonded tubings wherein the inner tubular portion is of a radiopaque polyvinyl chloride/silicone network polymer composition and wherein the outer shell is a clear polyurethane.

| | |
|---|---|
| <u>Inner Core (Silicone network polymer - 20%)</u> | |
| <u>PART A - Composition (parts by weight)</u> | |
| Resin, thermoplastic polyvinylchloride, BK 80[a] | 1800 |
| 2-Ethoxyethyl-2,5-diiodobenzoate | 600 |
| n-Butyl 2,3,4,6-tetraiodobenzoate | 600 |
| Plasticizer, Epoxol 9-5[b] | 160 |
| Heat stabilizer, CZ-11C[c] | 40 |
| Methylvinyl polysiloxane[d] | 800 |
| <u>PART B - Composition (parts by weight)</u> | |
| Resin, thermoplastic polyvinylchloride, BK 80 | 1800 |
| 2-Ethoxyethyl-2,5-diiodobenzoate | 600 |
| n-Butyl 2,3,4,6-tetraiodobenzoate | 600 |
| Plasticizer, Epoxol 9-5 | 160 |
| Heat stabilizer, CZ-11C | 40 |
| Methylhydrogen polysiloxane[e] | 800 |
| Platinum-olefin complex catalyst[f] | 0.080 |
| <u>Outer Shell</u> | |
| <u>Composition (parts by weight)</u> | |
| Resin, thermoplastic polyurethane, (Pellethane 75-80 D)[g] | 1800 |

a-f See Example 15, footnotes a-f.

g Upjohn Co.

<u>EXAMPLE 23</u>

The procedure of Example 1 is repeated, but the extruded tubing has an outer diameter of 2,45 mm (0.098 inch) and an inside diameter of 1,37 mm (0.054) inch. This is formed into a finished catheter as illustrated in FIG. 1. Tubing 10 tapers down to a main body section 11 of substantially uniform cross-section to a curved J-shaped tip 12 as its distal end 14. One or more eyes or openings 15 are also provided in the distal end 14 for passages of fluids through the tubing 10. The proximal end of tubing 10 flares outwardly for receiving various equipment such as syringes for injecting fluids into the patient. The tapering and curvature of the tip, the flaring of the eyes and the flaring of the end are all effected by heated tools which utilize the thermoplastic properties of the extruded tubing. This single wall tubing has a cross-section as in FIG. 2 where reference numeral 30 illustrates the radiopaque composition of this invention.

Unlike catheters made from wholly vinyl resins, the catheter of this example readily resumes its shape after stretching and deforming.

<u>EXAMPLE 24</u>

A double wall radiopaque medical-surgical tubing is extruded in the temperature range of 154,45 °C to 165,56 °C (310 °F. to 330 °F). in a conventional manner using conventional extrusion equipment having a biorifice tubular die for co-extrusion of an inner core and an outer shell bonded to said inner tube. The tubing has an outer diameter of 0.098 inch and an inner diameter of 1,37 mm (0.054 inch), and the inner tube has a thickness of 0,13 mm (0.005 inch) and the outer shell has a thickness of 0,43 mm (0.017 inch). It will be understood that the dimensions given are illustrative but not limiting.

Referring to FIG. 3, the thinner inner tube 18 comprises the two-part radiopaque thermoplastic polyurethane/silicone network composition of Example 1.

The thicker outer wall 20 comprises the following: 65 parts of poly(vinyl chloride) resin plasticized with 34 parts of epoxy plasticizer, Epoxol-9-5, stabilized with 1 part of calcium zinc stabilizer. This multiwall tubing is radiopaque of low cost construction, because of the P.V.C. heavy wall, and is especially useful in larger sizes for stomach and nasogastric tubes. There is no possibility for PVC or plasticizer to elute into the fluid path (shown as 19 in FIG. 3).

Similar advantages are provided when a tubing is constructed according to these examples, but using nylon, and polypropylene, instead of vinyl in outer wall 20 of FIG. 3.

EXAMPLES 25-26

Two multiwall tubes are made by the procedure of Example 24, except that the inner tube 34 (FIG. 4) comprises, nylon on the one hand, and polypropylene, on the other, and the outer shell 36 comprises the radiopaque two-part thermoplastic polyurethane/silicone network composition of Example 1. Each tubing provides catheters with superior torque characteristics in terms of resistance to kinking when passed through tortuous body passages.

EXAMPLES 27-28

Two co-tapered multiwall tubes are made by a modification of the procedure in Examples 25-26. The inner and outer layers are extruded concurrently and concentrically through annular concentric orifices of a multiorifice extruder so as to bond the inner and outer layers together. The co-tapering of the inner and outer layers is obtained by varying the rate of extrusion of the inner and outer layers. See the above-mentioned Burlis et al. patent and Flynn, U.S. 4,250,072. The rate of extrusion of the inner layer gradually varies from a first rate to a second rate while the rate of extrusion of the outer layer gradually varies inversely for the inner layer.

To exemplify further, reference is made to the accompanying drawing of FIG. 6. FIG. 6 is a schematic view of a bi-orifice extrusion head 52 from which tubing 40 is being extruded. Inner layer 60 is being discharged from the inner annular orifice of extrusion head 52. The thermoplastic composition for inner layer 60 is delivered to the extruder head 52 by extruder 56 at a decreasing, uniform rate. The outer layer 38 is being discharged from the concentric outer annular orifice of extruder head 52, the thermoplastic composition therefor being supplied by extruder 54 at an increasing rate inversely proportional to the declining rate of extruder 56. Since the tube 40 is being drawn by a Godet (not seen in FIG. 6) the inner layer 60 is tapering downward in thickness while the outer layer 38 is tapering upward in thickness. Air line 58 provides support for the thermoplastic walls 38, 60 and assists in defining lumen 42 during the extrusion. The increasing and decreasing rates of extrusion for inner and outer walls 60, 38 may be provided over any convenient length of time cycle to provide continuous lengths of co-tapered, multi-wall tubing of the invention. The rates of discharge from extruders 54 and 56 may then be reversed to begin the taper in the opposite direction, or the original first cycle may be repeated by synchronizing the speed of extrusion from the two extruders and then again increasing and decreasing the respective rates of discharge from each extruder 54, 56.

The co-tapered tubings are illustrated in FIG. 5 in one embodiment.

In Example 27, inner wall 60 (FIG. 5) comprises nylon and outer wall 18 comprises the radiopaque two-part thermoplastic polyurethane/silicone network of Example 1.

In Example 28, inner wall 60 (FIG. 5) comprises polypropylene and outerwall 18 comprises the radiopaque two-part thermoplastic polyurethane/silicone network of Example 1.

Surgical catheters are formed from the respective tubings of Examples 27 and 28, and they exhibit a degree of flexibility and softening progressively changing from one end to the other. Such medical-surgical catheters have manipulative characteristics which are advantageously employed by the surgical operator in inserting, withdrawing and controlling the catheter.

EXAMPLE 29

A multiwall tube of co-taper construction is made following the preceeding example where the inner core is softer than the outer jacket. This is uniquely advantageous wherein higher torque is desired.

In Example 29, softer inner core 60 (FIG. 5) comprises a 55 to 65D durometer radiopaque two-part thermoplastic polyurethane/silicone network polymer containing 20 parts per 100 parts by weight of composition of 2-ethoxyethyl 2,5-diiodobenzoate; and harder outerwall 18 comprises a 75D durometer radiopaque two-part thermoplastic polyurethane/silicone network polymer and 20 parts per 100 parts by weight of composition of 2-ethoxyethyl 2,5-diiodobenzoate.

| | |
|---|---|
| Inner Core | |
| Silicone-network polymer (20%) | |
| Part A - Composition (parts by weight) | |
| Resin, thermoplastic polyurethane (55-65 D durometer) | 1800 |
| Methylvinyl polysiloxane[a] | 200 |
| 2-Ethoxyethyl-2,5-diiodobenzoate | 500 |
| Part B - Composition (parts by weight) | |
| Resin, thermoplastic polyurethane (55-65 D durometer) | 1800 |
| Methylhydrogen polysiloxane[b] | 200 |
| Platinum-olefin complex catalyst[c] | .02 |
| 2-Ethoxyethyl-2,5-diiodobenzoate | 500 |
| Outer Core | |
| Silicone-network polymer (20%) | |
| Part A - Composition (parts by weight) | |
| Resin, thermoplastic polyurethane (75 D durometer) | 1800 |
| Methylvinyl polysiloxane[a] | 200 |
| 2-Ethoxyethyl-2,5-diiodobenzoate | 500 |
| Part B - Composition (parts by weight) | |
| Resin, thermoplastic polyurethane (75 D durometer) | 1800 |
| Methylhydrogen polysiloxane[b] | 200 |
| Platinum-ethylene complex catalyst[c] | .02 |
| 2-Ethoxyethyl-2,5-diiodobenzoate | 500 |

a-c See Example 1, footnote a-c

EXAMPLE 30

A thin wall tubing 0,13 mm (0.005") wall thickness) is extruded from the following compositions:

Part A

Silicone-network polymer (16.7%)

| Composition (parts by weight) | |
|---|---|
| Resin, thermoplastic polyurethane Rucothene 363 | 38.04 |
| Resin, poly(vinyl chloride) BK-80 Goodyear | 10.39 |
| n-Butyl 2,3,4,6-tetraiodobenzoate | 18.39 |
| 2-Ethoxyethyl 2,5-diiodobenzoate | 3.08 |
| Epoxy plasticizer, Epoxol 9-5 | 1.21 |
| Stabilizer CZ-11C | 0.17 |
| Bismuth oxychloride | 28.57 |
| Methylvinyl polysilozne[a] | 20.00 |

Part B

Silicone-network polymer (16.7%)

| Composition (parts by weight) | |
|---|---|
| Resin, thermoplastic polyurethane Rucothene 363 | 38.04 |
| Resin, poly(vinyl chloride) BK-80 Goodyear | 10.39 |
| n-Butyl 2,3,4,6-tetraiodobenzoate | 18.39 |
| 2-Ethoxyethyl 2,5-diiodobenzoate | 3.08 |
| Epoxy plasticizer, Epoxol 9-5 | 1.21 |
| Stabilizer CZ-11C | 0.17 |
| Bismuth oxychloride | 28.57 |
| Methylhydrogen polysiloxane[b] | 20.00 |
| Platinum-olefin complex catalyst[c] | 0.006 |

a-c See Example 1, footnote a-c.

In comparison with tubing without bismuth oxychloride, this tubing has an enhanced tensile strength and higher radiopacity. The procedure is repeated substituting equal parts by weight of barium sulfate per 100 parts by weight of the composition for the bismuth oxychloride. Again higher tensile strength and higher radiopacity are obtained.

Such tubes are especially useful to provide miniballoon catheters.

Obviously, the compositions of this invention provide tubings with many advantages. With respect to

polytetrafluoroethylene catheters, the present invention overcomes an inability to provide a range of soft to hard catheters in a variety of wall thicknesses. This leads to a reduction in mechanical trauma induced by stiffer catheters. Also polytetrafluoroethylene is limited in its ability to accept adequate loading of radiopaque media to be clearly visible, if a small section is lost or fragmented. Furthermore, both the urethanes and the urethane-vinyls can be co-extruded according to this invention with a hard vinyl or urethane liner, which on drawn down produces a thin, hard knife edge impervious to peel back or fragmentation seen with the polytetrafluoroethylene catheters.

With respect to prior art vinyl tubings, drug vehicle compatibility with the present tubings is extended in numbers and concentration, and iodine extraction reduce, because of the ability of the urethanes and PVC-urethanes to lock in the iodo-esters. For example, they exhibit no spew or exudation when tested for four days at 100% humidity.

## Claims

**1.** A radiopaque composition comprising:

(a) a resin which includes from 100 to 0 parts by weight of a thermoplatic polyurethane and correspondingly from 0 to 100 parts of a polymer of a halogenated vinyl monomer per 100 parts by weight of resin;

(b) a radiopacifier therefor consisting of a diiodobenzoate or a tetraiodobenzoate compound of formula (I) or a triiodobenzoate of formula (II):

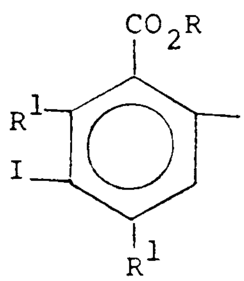

(I)

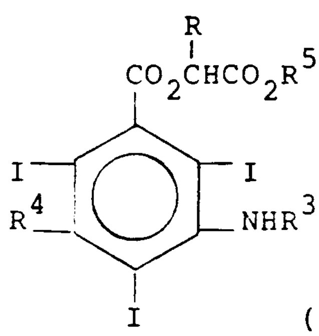

(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b), and, uniformly dispersed therein;

(c) a platinum-cured silicone network polymer, the amount of (c) comprising 8 to 30 parts by weight per 100 parts by weight of (a), (b) and (c), wherein said radiopacifier composition is essentially free of filler.

**2.** A composition as defined in Claim 1 wherein, in said radiopacifier (b) (I), each $R^1$ is iodo and R is (lower)alkyl or (lower)alkoxy(lower)alkyl.

**3.** A composition as defined in Claim 1 wherein said radiopacifier (b) (I) is n-butyl 2,3,4,6-tetraiodobenzoate.

**4.** A composition as defined in Claim 1 wherein said radiopacifier (b) (I) is 2-ethoxyethyl 2,5-diiodobenzoate.

**5.** A composition as defined in Claim 1 wherein said radiopacifier (b) (I) is a mixture of n-butyl 2,3,4,6-tetraiodobenzoate and 2-ethyoxyethyl 2,5-diiodobenzoate.

**6.** A composition as defined in Claim 1 wherein said radiopacifier (b) (II) is ethyl (3-amino-2,4,6-triiodobenzoyloxy)acetate.

**7.** A composition as defined in Claim 1 wherein said radiopacifier (b) (II) is ethyl 2-(3-amino-2,4,6-triiodobenzoyloxy)butanoate.

**8.** A composition as defined in Claim 1 which is essentially free of particulate silica.

**9.** A composition as defined in Claim 1 wherein resin component (a) comprises from 100 to 33 parts by weight of thermoplastic polyurethane and correspondingly from 0 to 67 parts by weight of a polymer of a halogenated vinyl monomer per 100 parts by weight of resin.

**10.** A composition as defined in Claim 1 wherein resin component (a) comprises a vinyl resin which is a polymer or copolymer of a halogenated vinyl monomer.

**11.** A composition as defined in Claim 1 wherein said platinum-cured silicone network polymer (c) comprises the reaction product of
(i) an organic silicon compound having at least two silicon-bonded unsaturated groups per molecule,
(ii) a silicon compound containing at least two silicon-bonded hydrogen atoms per molecule, and
(iii) a small, catalytically effective amount of a platinum-containing catalyst.

**12.** A composition as defined in Claim 11 wherein component (i) is of the formula:

$$(R^6)_a(R^7)_b\ SiO_{\frac{4-a-b}{2}} \quad ;$$

and component (ii) is of the formula:

$$(R^6)_a(H)_b\ SiO_{\frac{4-a-b}{2}}$$

where $R^6$ is selected from monovalent hydrocarbon radicals free of olefinic unsaturation, halogenated monovalent hydrocarbon radicals free of olefinic unsaturation, cyanoalkyl radicals or a mxture of any of the foregoing; $R^7$ is selected from vinyl radicals, allyl radicals or a mixture thereof; a has a value of from 1.0 to 2.5; b has a value of from 0.005 to 2.0; and the sum of a plus b is equal to from 1.005 to 3.0.

**13.** A composition as defined in Claim 11 wherein component (iii) is selected from chloroplatinic acid, platinum black, platinum adsorbed on a carrier, a platinum-olefin complex, a platinum-cyclopropane complex or a mixture of any of the foregoing.

**14.** A two part composition adapted for melt blending and thermoforming into radiopaque tubing,
Part A comprising:
(a) a resin which includes from 100 to 0 parts by weight of a thermoplastic polyurethane and, correspondingly, from 0 to 100 parts of a polymer of halogenated vinyl monomer per 100 parts by weight of resin;
(b) a radiopacifier therefor consisting of a diiodobenzoate or a tetraiodobenzoate compound of formula (I) or a triiodobenzoate of formula (II):

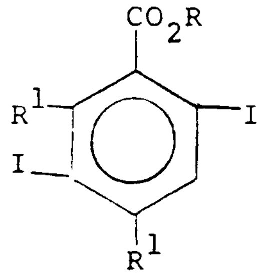
(I)

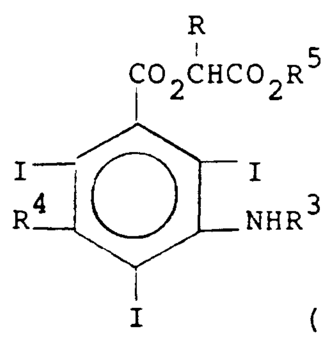
(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b), and

(c) (i) an organic silicon compound having at least two silicon-bonded unsaturated groups per molecule; and

Part B comprising:

(a) a resin which includes from 100 to 0 parts by weight of a thermoplastic polyurethane and, correspondingly, from 0 to 100 parts of a polymer of a halogenated vinyl monomer per 100 parts of weight of resin;

(b) a radiopacifier therefor consisting of a diiodobenzoate or a tetraiodobenzoate compound of the formula:

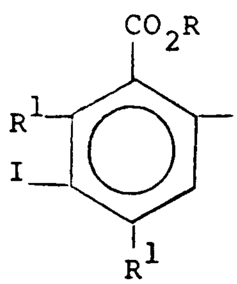
(I)

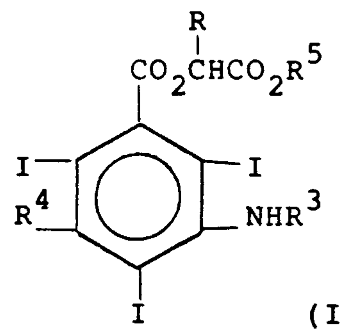
(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b), and;

(c) (ii) a silicon compound containing at least two silicon-bonded hydrogen atoms per molecule; and

(c)(iii) a small, catalytically effective amount of a platinum-containing catalyst, wherein the respective amounts of (c)(i), (ii) and (iii) are selected to provide from about 8 to about 30 parts by weight of a platinum-cured silicone network polymer, after having blended and thermoformed parts A and B, per 100 parts by weight of the total composition, and wherein said two part composition is essentially free of filler.

**15.** A two-part composition as defined in Claim 14 wherein, in said radiopacifier (b) (I), each $R^1$ is iodo and R is (lower)alkyl or (lower)alkoxy(lower)alkyl.

**16.** A two-part composition as defined in Claim 14 wherein said radiopacifier (b) (I) and (b) (II) is selected from n-butyl 2,3,4,6-tetraiodobenzoate, 2-ethoxyethyl 2,5-diiodobenzoate, ethyl (3-amino-2,4,6-triiodobenzoyloxy)acetate, ethyl 2-(3-amino-2,4,6-triiodobenzoyloxy)butanoate or a mixture of the foregoing.

**17.** A two-part composition as defined in Claim 14 which is essentially free of particulate silica.

**18.** A two-part composition as defined in Claim 14 wherein component (c) (i) of Part A is of the formula:

$$(R^6)_a(R^7)_b\,SiO_{\frac{4-a-b}{2}} \quad ;$$

and component (c) (ii) of Part B is of the formula:

$$(R^6)_a(H)_b\,SiO_{\frac{4-a-b}{2}}$$

where $R^6$ is selected from monovalent hydrocarbon radicals free of olefinic unsaturation, halogenated monovalent hydrocarbon radicals free of olefinic unsaturation and cyanoalkyl radical or a mixture of any of the foregoing; $R^7$ is selected from vinyl radicals, allyl radicals or a mixture thereof; a has a value of from 1.0 to 2.5; b has a value of from 0.005 to 2.0; and the sum of a plus b is equal to from 1.005 to 3.0.

**19.** A two-part composition as defined in Claim 18 wherein component (c) (iii) is selected from chloroplatinic acid, platinum black, platinum adsorbed on a carrier, a platinum-olefin complex, a platinum-cyclopropane complex or a mixture of any of the foregoing.

**20.** Medical-surgical tubing comprising:

(a) a resin which includes from 100 to 0 parts by weight of a thermoplastic polyurethane and correspondingly from 0 to 100 parts of a polymer of a halogenated vinyl monomer per 100 parts by weight of resin;

(b) a radiopacifier therefor consisting of a diiodobenzoate or a tetraiodobenzoate compound of formula (I) or a triiodobenzoate of formula (II):

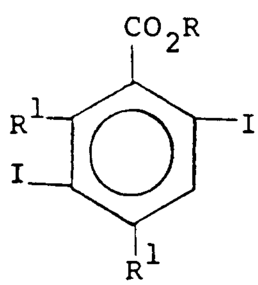
(I)

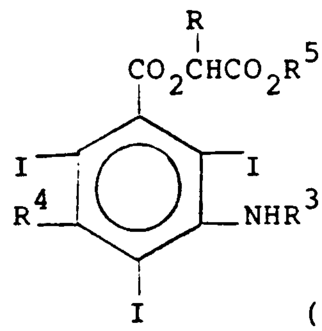
(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b), and, uniformly dispersed therein;

(c) a platinum-cured silicone network polymer, the amount of (c) comprising 8 to 30 parts by weight per 100 parts by weight of (a), (b) and (c), wherein said medical-surgical tubing is essentially free of filler.

**21.** Medical-surgical tubing as defined in Claim 20 wherein, in said radiopacifier (b) (I), each $R^1$ is iodo and R is (lower)alkyl or (lower)alkoxy(lower)alkyl.

**22.** Medical-surgical tubing as defined in Claim 20 wherein said radiopacifier (b) (I) is n-butyl 2,3,4,6-tetraiodobenzoate.

**23.** Medical-surgical tubing as defined in Cliam 20 wherein said radiopacifier (b) (I) is 2-ethoxyethyl 2,5-diiodobenzoate.

**24.** Medical-surgical tubing as defined in Claim 20 wherein said radiopacifier (b) is a mixture of n-butyl 2,3,4,6-tetraiodobenzoate and 2-ethoxyethyl 2,5-diiodobenzoate.

**25.** Medical-surgical tubing as defined in Claim 20 wherein said radiopacifier (b)(II) is ethyl(3-amino-2,4,6-triiodobenzoyloxy)acetate.

**26.** Medical-surgical tubing as defined in Claim 20 wherein said radiopacifier (b)(II) is ethyl 2-(3-amino-2,4,6-triiodobenzoyloxy)butanoate.

**27.** Medical-surgical tubing as defined in Claim 20 wherein resin component (a) comprises from 100 to 33 parts by weight of thermoplastic polyurethane and correspondingly from 0 to 67 parts by weight of a polymer of a halogenated vinyl monomer per 100 parts by weight of resin.

**28.** Medical-surgical tubing as defined in Claim 20 wherein resin component (a) comprises a vinyl resin which is a polymer or copolymer of a halogenated vinyl monomer.

**29.** Medical-surgical tubing as defined in Claim 20 which is essentially free of particulate silica.

**30.** Medical-surgical tubing as defined in Claim 20 wherein said platinum-cured silicone network polymer (c) comprises the reaction product of:
(i) an organic silicon compound having at least two silicon-bonded unsaturated groups per molecule,
(ii) a silicon compound containing at least two silicon-bonded hydrogen atoms per molecule, and
(iii) a small, catalytically effective amount of a platinum-containing catalyst.

**31.** Medical-surgical tubing as defined in Claim 31 wherein component (i) is of the formula:

$$(R^6)_a(R^7)_b\ SiO_{\frac{4-a-b}{2}} \quad ;$$

and component (ii) is of the formula:

$$(R^6)_a(H)_b\ SiO_{\frac{4-a-b}{2}}$$

where $R^6$ is selected from monovalent hydrocarbon radicals free of olefinic unsaturation, halogenated monovalent hydrocarbon radicals free of olefinic unsaturation, cyanoalkyl radicals or a mixture of any of the foregoing; $R^7$ is selected from vinyl radicals, allyl radicals or a mixture thereof; a has a value of from 1.0 to 2.5; b has a value of from 0.005 to 2.0; and the sum of a plus b is equal to from 1.005 to 3.0.

**32.** Medical-surgical tubing as defined in Claim 31 wherein component (iii) is selected from chloroplatinic acid, platinum black, platinum adsorbed on a carrier, a platinum-olefin complex, a platinum-cyclopropane complex or a mixture of any of the foregoing.

**33.** Medical-surgical tubing comprising a plurality of concentric tubular portions, at least one of which comprises a radiopaque composition as defined in Claim 1.

**34.** Medical-surgical tubing comprising a plurality of concentric tubular portions, at least one of which comprises a radiopaque composition as defined in Claim 1 and at least one of which is visually transparent and transparent to x-ray radiation.

**35.** A method for the production of radiopaque medical-surgical tubing, said method comprising:
(1) providing a two-part composition which is essentially free of filler, Part A comprising:
(a) a resin which includes from 100 to 0 parts by weight of a thermoplastic polyurethane and correspondingly from 0 to 100 parts of a polymer of a halogenated vinyl monomer per 100 parts by weight of resin;
(b) a radiopacifier therefor consisting of a diiodobenzoate or a tetraiodobenzoate compound of formula (I) or a triiodobenzoate of formula (II):

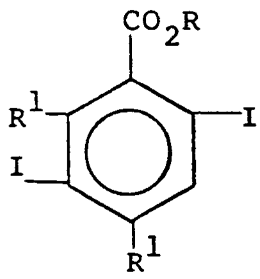

(I)

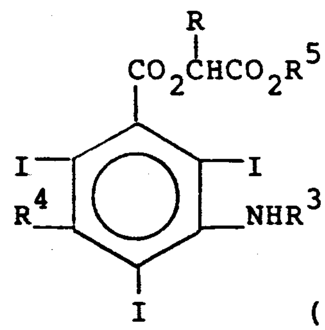

(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b); and (c)(i) a silicon compound containing at least two silicon-bonded unsaturated groups per molecule; and

Part B comprising:

(a) a resin which includes from 100 to 0 parts by weight of a thermoplastic polyurethane and correspondingly from 0 to 100 parts of a polymer of a halogenated vinyl monomer per 100 parts by weight of resin;

(b) a radiopacifier therefor consisting of a diiodobenzoate or a tetraiodobenzoate compound of formula (I) or a triiodobenzoate of formula (II):

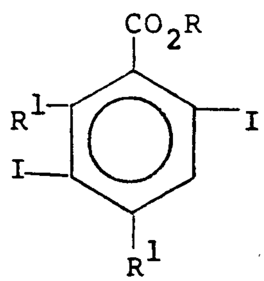

(I)

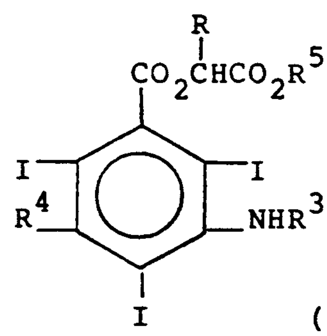

(II)

wherein R is alkyl or alkoxyalkyl, $R^1$ is hydrogen or iodo, $R^2$ is hydrogen or alkyl, $R^3$ is hydrogen or alkanoyl, $R^4$ is hydrogen or 1 alkanoylamino, and $R^5$ is alkyl or a mixture of said compounds, said radiopacifier comprising 10 to 40 parts by weight per 100 parts by weight of (a) and (b); and (c)(ii) a silicon compound containing at least two silicon-bonded hydrogen atoms per molecule; and (c) (iii) a small, catalytically effective amount of a platinum-containing catalyst, the respective amounts of (c)(i), (ii) and (iii) being selected to provide from about 8 to about 30 parts by weight of a platinum-silicone network polymer, per 100 parts by weight of the total composition;

(2) melt blending Part A and Part B, and

(3) extruding the blended mixture of (2) into tubing.

## Revendications

**1.** Composition radio-opaque, comprenant :

(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;

(b) un opacifiant radiologique pour cette résine, consistant en un di-iodobenzoate ou un tétra-iodobenzoate, de formule (I), ou en un tri-iodobenzoate de formule (II) :

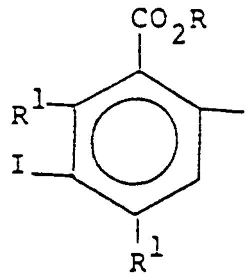
(I)

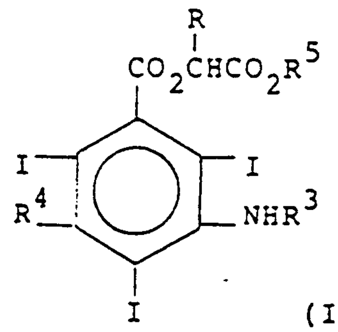
(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,
ou en un mélange de tels composés, ledit opacifiant radiologique étant présent à raison de 10 à 40 parties en poids pour 100 parties en poids de (a) et (b), et, dispersé là-dedans de façon homogène,
(c) un polymère réticulé de type silicone, durci au platine, la quantité de (c) représentant de 8 à 30 parties en poids pour 100 parties en poids de (a), (b) et (c), ladite composition d'opacifiant radiologique étant pratiquement exempte de charge.

**2.** Composition telle que définie dans la revendication 1, dans laquelle, dans ledit opacifiant radiologique (b) (I), chaque $R^1$ représente un atome d'iode et R représente un groupe alkyle inférieur ou un groupe (alcoxy inférieur)-alkyle inférieur.

**3.** Composition telle que définie dans la revendication 1, dans laquelle ledit opacifiant radiologique (b) (I) est du 2,3,4,6-tétra-iodobenzoate de n-butyle.

**4.** Composition telle que définie dans la revendication 1, dans laquelle ledit opacifiant radiologique (b) (I) est du 2,5-di-iodobenzoate de 2-éthoxyéthyle.

**5.** Composition telle que définie dans la revendication 1, dans laquelle ledit opacifiant radiologique (b) (I) est un mélange de 2,3,4,6-tétra-iodobenzoate de n-butyle et de 2,5-di-iodobenzoate de 2-éthoxyéthyle.

**6.** Composition telle que définie dans la revendication 1, dans laquelle ledit opacifiant radiologique (b) (II) est du (3-amino-2,4,6-triiodobenzoyloxy)-acétate d'éthyle.

**7.** Composition telle que définie dans la revendication 1, dans laquelle ledit opacifiant radiologique (b) (II) est du 2-(3-amino-2,4,6-triiodobenzoyloxy)-butanoate d'éthyle.

**8.** Composition telle que définie dans la revendication 1, qui est pratiquement exempte de particules de silice.

**9.** Composition telle que définie dans la revendication 1, dans laquelle le composant résine (a) est constitué de 100 à 33 parties en poids d'un polyuréthane thermoplastique et, de façon correspondante, de 0 à 67 parties en poids d'un polymère d'un monomère vinylique halogéné, pour 100 parties en poids de résine.

**10.** Composition telle que définie dans la revendication 1, dans laquelle le composant résine (a) comporte une résine vinylique qui est un polymère ou un copolymère d'un monomère vinylique halogéné.

**11.** Composition telle que définie dans la revendication 1, dans laquelle ledit polymère réticulé (c) de type silicone, durci au platine, comprend le produit de réaction de
(i) un composé organique du silicium comportant au moins deux groupes insaturés liés à du silicium par molécule,
(ii) un composé du silicium comportant au moins deux atomes d'hydrogène liés à du silicium par molécule, et

(iii) une faible quantité, efficace du point de vue catalytique, d'un catalyseur contenant du platine.

**12.** Composition telle que définie dans la revendication 11, dans laquelle le composant (i) présente la formule :

$$(R^6)_a(R^7)_bSiO_{\frac{4-a-b}{2}} \quad ;$$

et le composant (ii) présente la formule :

$$(R^6)_a(H)_bSiO_{\frac{4-a-b}{2}}$$

dans lesquelles formules $R^6$ est choisi parmi les groupes hydrocarbonés monovalents ne présentant pas d'insaturation oléfinique, les groupes hydrocarbonés monovalents halogénés ne présentant pas d'insaturation oléfinique, les groupes cyanoalkyles ou un mélange de n'importe lesquels des groupes mentionnés ci-dessus, $R^7$ est choisi parmi les groupes vinyliques, les groupes allyliques ou un de leurs mélanges, a vaut de 1,0 à 2,5, b vaut de 0,005 à 2,0 et la somme de a et de b vaut de 1,005 à 3,0.

**13.** Composition telle que définie dans la revendication 11, dans laquelle le composant (iii) est choisi parmi l'acide chloroplatinique, le noir de platine, du platine adsorbé sur un support, un complexe de platine et d'oléfine, un complexe de platine et de cyclopropane ou un mélange de n'importe lesquels des composants mentionnés ci-dessus.

**14.** Composition en deux parties, adaptée pour un mélange à l'état fondu et une mise sous forme d'un tuyau radio-opaque sous l'action de la chaleur, dont une partie A comprend :

(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;

(b) un opacifiant radiologique pour cette résine, consistant en un di-iodobenzoate ou un tétra-iodobenzoate, de formule (I), ou en un tri-iodobenzoate de formule (II) :

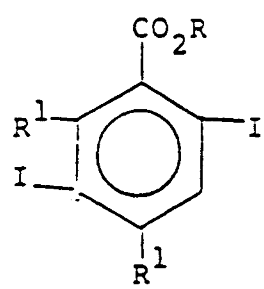
(I)

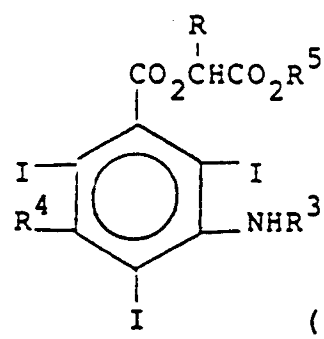
(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,

ou en un mélange de tels composés, ledit opacifiant radiologique étant présent à raison de 10 à 40 parties en poids pour 100 parties en poids de (a) et (b), et

(c)(i) un composé organique du silicium comportant au moins deux groupes insaturés liés à du silicium par molécule ;

et dont l'autre partie B comprend :

(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;

(b) un opacifiant radiologique pour cette résine, consistant en un di-iodobenzoate ou un tétra-iodobenzoate, de formule (I), ou en un tri-iodobenzoate de formule (II) :

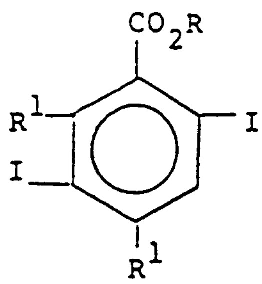
(I)

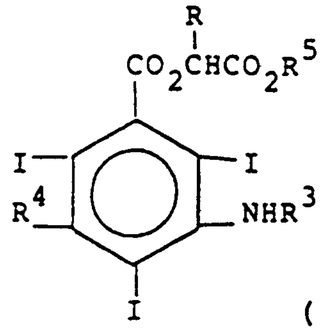
(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,

ou en un mélange de tels composés, ledit opacifiant radiologique étant présent à raison de 10 à 40 parties en poids pour 100 parties en poids de (a) et (b), et,

(c)(ii) un composé du silicium comportant au moins deux atomes d'hydrogène liés à du silicium par molécule, et

(c)(iii) une faible quantité, efficace du point de vue catalytique, d'un catalyseur contenant du platine, et les quantités respectives de (c)(i), (ii) et (iii) sont choisies de façon à fournir d'environ 8 à environ 30 parties en poids de polymère réticulé de type silicone, durci au platine, après que l'on a mélangé et mis en forme, sous l'action de la chaleur, les parties A et B, pour 100 parties en poids de la composition totale, et ladite composition en deux parties ne contenant pratiquement pas de charge.

**15.** Composition en deux parties telle que définie dans la revendication 14, dans laquelle, dans ledit opacifiant radiologique (b)(I), chaque $R^1$ représente un atome d'iode et R représente un groupe alkyle inférieur ou un groupe (alcoxy inférieur)-alkyle inférieur.

**16.** Composition en deux parties telle que définie dans la revendication 14, dans laquelle ledit opacifiant radiologique (b)(I) et (b)(II) est choisi parmi le 2,3,4,6-tétra-iodobenzoate de n-butyle, le 2,5-di-iodobenzoate de 2-éthoxyéthyle, le (3-amino-2,4,6-tri-iodo-benzoyloxy)-acétate d'éthyle, le 2-(3-amino-2,4,6-tri-iodobenzoyloxy)-butanoate d'éthyle et un mélange des composés précédents.

**17.** Composition en deux parties telle que définie dans la revendication 14, qui ne contient pratiquement pas de particules de silice.

**18.** Composition en deux parties telle que définie dans la revendication 11, dans laquelle le composant (c)-(i) de la partie A présente la formule :

$$(R^6)_a(R^7)_bSiO_{\frac{4-a-b}{2}} \quad ;$$

et le composant (c)(ii) de la partie B présente la formule :

$$(R^6)_a(H)_bSiO_{\frac{4-a-b}{2}}$$

dans lesquelles formules $R^6$ est choisi parmi les groupes hydrocarbonés monovalents ne présentant pas d'insaturation oléfinique, les groupes hydrocarbonés monovalents halogénés ne présentant pas

d'insaturation oléfinique, les groupes cyanoalkyles ou un mélange de n'importe lesquels des groupes mentionnés ci-dessus, $R^7$ est choisi parmi les groupes vinyliques, les groupes allyliques ou un de leurs mélanges, a vaut de 1,0 à 2,5, b vaut de 0,005 à 2,0 et la somme de a et de b vaut de 1,005 à 3,0.

**19.** Composition en deux parties telle que définie dans la revendication 18, dans laquelle le composant (c)-(iii) est choisi parmi l'acide chloroplatinique, le noir de platine, du platine adsorbé sur un support, un complexe de platine et d'oléfine, un complexe de platine et de cyclopropane ou un mélange de n'importe lesquels des composants mentionnés ci-dessus.

**20.** Tuyau médico-chirurgical, comprenant :

(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;

(b) un opacifiant radiologique pour cette résine, consistant en un di-iodobenzoate ou un tétra-iodobenzoate, de formule (I), ou en un tri-iodobenzoate de formule (II) :

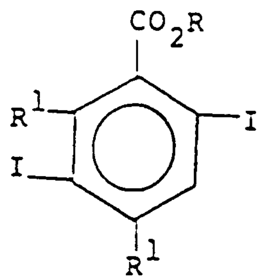
(I)

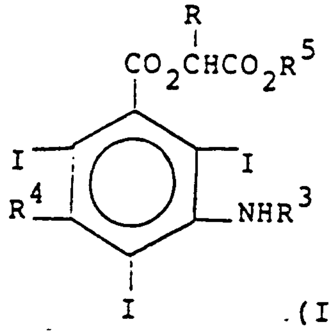
.(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,

ou en un mélange de tels composés, ledit opacifiant radiologique étant présent à raison de 10 à 40 parties en poids pour 100 parties en poids de (a) et (b), et, dispersé là-dedans de façon homogène,

(c) un polymère réticulé de type silicone, durci au platine, la quantité de (c) représentant de 8 à 30 parties en poids pour 100 parties en poids de (a), (b) et (c), ladite composition d'opacifiant radiologique étant pratiquement exempte de charge.

**21.** Tuyau médico-chirurgical tel que défini dans la revendication 20, dans lequel, dans ledit opacifiant radiologique (b) (I), chaque $R^1$ représente un atome d'iode et R représente un groupe alkyle inférieur ou un groupe (alcoxy inférieur)-alkyle inférieur.

**22.** Tuyau médico-chirurgical tel que défini dans la revendication 20, dans lequel ledit opacifiant radiologique (b) (I) est du 2,3,4,6-tétra-iodobenzoate de n-butyle.

**23.** Tuyau médico-chirurgical tel que défini dans la revendication 20, dans lequel ledit opacifiant radiologique (b) (I) est du 2,5-di-iodobenzoate de 2-éthoxyéthyle.

**24.** Tuyau médico-chirurgical tel que défini dans la revendication 20, dans lequel ledit opacifiant radiologique (b) est un mélange de 2,3,4,6-tétra-iodobenzoate de n-butyle et de 2,5-di-iodobenzoate de 2-éthoxyéthyle.

**25.** Tuyau médico-chirurgical tel que défini dans la revendication 20, dans lequel ledit opacifiant radiologique (b)(II) est du (3-amino-2,4,6-tri-iodobenzoyloxy)-acétate d'éthyle.

**26.** Tuyau médico-chirurgical tel que défini dans la revendication 20, dans lequel ledit opacifiant radiologique (b) (II) est du 2-(3-amino-2,4,6-tri-iodobenzoyloxy)-butanoate d'éthyle.

**27.** Tuyau médico-chirurgical tel que défini dans la revendication 20, dans lequel le composant résine (a) est constitué de 100 à 33 parties en poids d'un polyuréthane thermoplastique et, de façon correspondante, de 0 à 67 parties en poids d'un polymère d'un monomère vinylique halogéné, pour 100 parties en poids de résine.

**28.** Tuyau médico-chirurgical tel que défini dans la revendication 20, dans lequel le composant résine (a) comporte une résine vinylique qui est un polymère ou un copolymère d'un monomère vinylique halogéné.

**29.** Tuyau médico-chirurgical tel que défini dans la revendication 20, qui est pratiquement exempte de particules de silice.

**30.** Tuyau médico-chirurgical tel que défini dans la revendication 20, dans lequel ledit polymère réticulé (c) de type silicone, durci au platine, comprend le produit de réaction de
(i) un composé organique du silicium comportant au moins deux groupes insaturés liés à du silicium par molécule,
(ii) un composé du silicium comportant au moins deux atomes d'hydrogéne liés à du silicium par molécule, et
(iii) une faible quantité, efficace du point de vue catalytique, d'un catalyseur contenant du platine.

**31.** Tuyau médico-chirurgical tel que défini dans la revendication 30, dans lequel le composant (i) présente la formule :

$$(R^6)_a(R^7)_bSiO_{\frac{4-a-b}{2}} \quad ;$$

et le composant (ii) présente la formule :

$$(R^6)_a(H)_bSiO_{\frac{4-a-b}{2}}$$

dans lesquelles formules $R^6$ est choisi parmi les groupes hydrocarbonés monovalents ne présentant pas d'insaturation oléfinique, les groupes hydrocarbonés monovalents halogénés ne présentant pas d'insaturation oléfinique, les groupes cyanoalkyles ou un mélange de n'importe lesquels des groupes mentionnés ci-dessus, $R^7$ est choisi parmi les groupes vinyliques, les groupes allyliques ou un de leurs mélanges, a vaut de 1,0 à 2,5, b vaut de 0,005 à 2,0 et la somme de a et de b vaut de 1,005 à 3,0.

**32.** Tuyau médico-chirurgical tel que défini dans la revendication 31, dans lequel le composant (iii) est choisi parmi l'acide chloroplatinique, le noir de platine, du platine adsorbé sur un support, un complexe de platine et d'oléfine, un complexe de platine et de cyclopropane ou un mélange de n'importe lesquels des composants mentionnés ci-dessus.

**33.** Tuyau médico-chirurgical, comprenant plusieurs parties tubulaires concentriques, dont l'une au moins est constituée par une composition radio-opaque telle que définie dans la revendication 1.

**34.** Tuyau médico-chirurgical, comprenant plusieurs parties tubulaires concentriques, dont l'une au moins est constituée d'une composition radio-opaque telle que définie dans la revendication 1 et dont une autre au moins est transparente à la vue et transparente aux rayons X.

**35.** Procédé pour la production d'un tuyau médico-chirurgical radio-opaque, ledit procédé comprenant :
(1) le fait de prendre une composition en deux parties, ne contenant pratiquement pas de charge, dont une partie A comprend :
(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;
(b) un opacifiant radiologique pour cette résine, consistant en un di-iodobenzoate ou un tétra-iodobenzoate, de formule (I), ou en un tri-iodobenzoate de formule (II) :

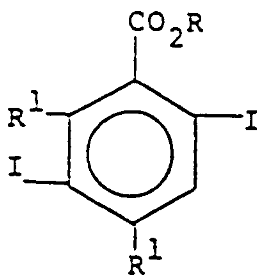
(I)

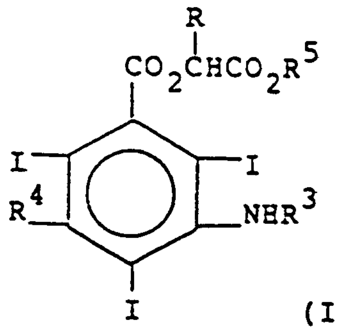
(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,

ou en un mélange de tels composés, ledit opacifiant radiologique étant présent à raison de 10 à 40 parties en poids pour 100 parties en poids de (a) et (b), et,

(c)(i) un composé organique du silicium comportant au moins deux groupes insaturés liés à du silicium par molécule ;

et dont l'autre partie B comprend :

(a) une résine qui contient de 100 à 0 parties en poids d'un polyuréthane thermoplastique et, de façon correspondante, de 0 à 100 parties d'un polymère d'un monomère vinylique halogéné, pour 100 parties en poids de résine ;

(b) un opacifiant radiologique pour cette résine, consistant en un di-iodobenzoate ou un tétra-iodobenzoate, de formule (I), ou en un tri-iodobenzoate de formule (II) :

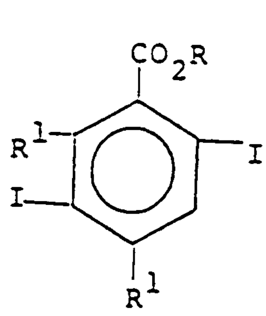
(I)

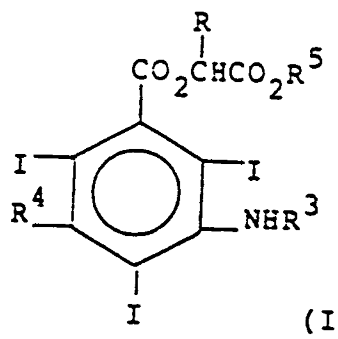
(II)

dans lesquelles formules R représente un groupe alkyle ou alkoxyalkyle, $R^1$ représente un atome d'hydrogène ou d'iode, $R^2$ représente un atome d'hydrogène ou un groupe alkyle, $R^3$ représente un atome d'hydrogène ou un groupe alcanoyle, $R^4$ représente un atome d'hydrogène ou un groupe alcanoylamino, et $R^5$ représente un groupe alkyle,

ou en un mélange de tels composés, ledit opacifiant radiologique étant présent à raison de 10 à 40 parties en poids pour 100 parties en poids de (a) et (b), et,

(c)(ii) un composé du silicium comportant au moins deux atomes d'hydrogène liés à du silicium par molécule, et

(c)(iii) une faible quantité, efficace du point de vue catalytique, d'un catalyseur contenant du platine,

et les quantités respectives de (c)(i), (ii) et (iii) étant choisies de façon à fournir d'environ 8 à environ 30 parties en poids de polymère réticulé de type silicone, durci au platine, après que l'on a mélangé et mis en forme, sous l'action de la chaleur, les parties A et B, pour 100 parties en poids de la composition totale ;

(2) le mélange à l'état fondu des parties A et B, et

(3) l'extrusion du mélange obtenu en (2) en un tuyau.

## Patentansprüche

1. Radioopake Zusammensetzung, umfassend:
(a) ein Harz, welches 100 bis 0 Gewichtsteile eines thermoplastischen Polyurethans und dementsprechend O bis 100 Teile eines Polymers eines halogenierten Vinylmonomers je 100 Gewichtsteile Harz umfaßt;
(b) ein Röntgenkontrastmittel hiefür, bestehend aus einer Diiodbenzoat-oder einer Tetraiodbenzoatverbindung der Formel (I) oder einem Triiodbenzoat der Formel (II):

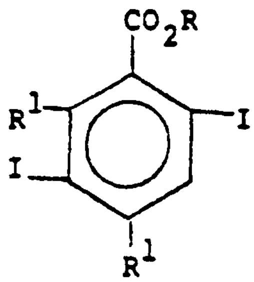

(I)

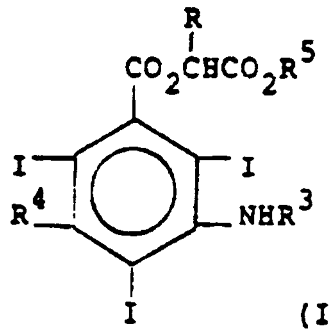

(II),

worin
R für Alkyl oder Alkoxyalkyl steht,
$R^1$ Wasserstoff oder Iod bedeutet,
$R^2$ Wasserstoff oder Alkyl darstellt,
$R^3$ Wasserstoff oder Alkanoyl bedeutet,
$R^4$ für Wasserstoff oder Alkanoylamino steht und
$R^5$ Alkyl darstellt,
oder einem Gemisch dieser Verbindungen,
welches Röntegenkontrastmittel 10 bis 40 Gewichtsteile je 100 Gewichtsteile von (a) und (b) ausmacht, und, gleichmäßig darin verteilt,
(c) ein Platin-gehärtetes Siliconnetzwerkpolymer, wobei die Menge von (c) 8 bis 30 Gewichtsteile je 100 Gewichtsteile von (a), (b) und (c) ausmacht, wobei diese Röntgenkontrastmittelzusammensetzung von Füllstoffen im wesentlichen frei ist.

2. Zusammensetzung nach Anspruch 1, worin in dem Röntgenkontrastmittel (b) (I) jeder Rest $R^1$ für Iod steht und R (Nieder)alkyl oder (Nieder)alkoxy(nieder)alkyl bedeutet.

3. Zusammensetzung nach Anspruch 1, worin das Röntgenkontrastmittel (b) (I) n-Butyl-2,3,4,6-tetraiodbenzoat ist.

4. Zusammensetzung nach Anspruch 1, worin das Röntgenkontrastmittel (b) (I) 2-Ethoxyethyl-2,5-diiodbenzoat ist.

5. Zusammensetzung nach Anspruch 1, worin das Röntgenkontrastmittel (b) (I) ein Gemisch aus n-Butyl-2,3,4,6-tetraiodbenzoat und 2-Ethoxyethyl-2,5-diiodbenzoat ist.

6. Zusammensetzung nach Anspruch 1, worin das Röntgenkontrastmittel (b) (II) Ethyl-(3-amino-2,4,6-triiodbenzoyloxy)acetet ist.

7. Zusammensetzung nach Anspruch 1, worin das Röntgenkontrastmittel (b) (II) Ethyl-2-(3-amino-2,4,6-triiodbenzoyloxy)butanoat ist.

8. Zusammensetzung nach Anspruch 1, welche im wesentlichen frei von teilchenförmigem Siliziumdioxid ist.

9. Zusammensetzung nach Anspruch 1, worin die Harzkomponente (a) 100 bis 33 Gewichtsteile thermoplastisches Polyurethan und dementsprechend 0 bis 67 Gewichtsteile eines Polymers eines halogenierten Vinylmonomers je 100 Gewichtsteile Harz umfaßt.

**10.** Zusammensetzung nach Anspruch 1, worin die Harzkomponente (a) ein Vinylharz umfaßt, das ein Polymer oder Copolymer eines halogenierten Vinylmonomers ist.

**11.** Zusammensetzung nach Anspruch 1, worin das Platin-gehärtete Siliconnetzwerkpolymer (c) das Reaktionsprodukt aus

(i) einer organischen Siliziumverbindung mit wenigstens zwei an Silizium gebundene ungesättigten Gruppen je Molekül,

(ii) einer Siliziumverbindung mit einem Gehalt an wenigstens zwei an Silizium gebundenen Wasserstoffatomen je Molekül und

(iii) einer Kleinen, katalytisch wirksamen Menge eines Platin-enthaltenden Katalysators umfaßt.

**12.** Zusammensetzung nach Anspruch 11, worin die Komponente (i) der Formel:

$$(R^6)_a(R^7)_b\ SiO_{\frac{4-a-b}{2}}$$

entspricht;
und die Komponente (ii) der Formel:

$$(R^6)_a(H)_b\ SiO_{\frac{4-a-b}{2}}$$

entspricht, wobei

$R^6$ unter einwertigen, von olefinischer Unsättigung freien Kohlenwasserstoffresten, halogenierten, von olefinischer Unsättigung freien einwertigen Kohlenwasserstoffresten, Cyanoalkylresten oder einem Gemisch der vorstehenden Reste ausgewählt ist;
$R^7$ unter Vinylresten, Allylresten oder einem Gemisch hievon ausgewählt ist;
a einen Wert von 1,0 bis 2,5 aufweist;
b einen Wert von 0,005 bis 2,0 aufweist;
und die Summe aus a plus b gleich 1,005 bis 3,0 beträgt.

**13.** Zusammensetzung nach Anspruch 11, worin die Komponente (iii) unter Chlorplatinsäure, Platinschwarz, auf einem Träger absorbiertem Platin, einem Platin-Olefin-Komplex, einem Platin-Cyclopropan-Komplex oder einem beliebigen Gemisch der vorstehend Genannten ausgewählt ist.

**14.** Zweiteilige, zum Schmelzmischen und Thermoformen zu radioopaken Schläuchen geeignete Zusammensetzung, worin der Teil A

(a) ein Harz, welches 100 bis 0 Gewichtsteile eines thermoplastischen Polyurethans und dementsprechend von 0 bis 100 Teile eines Polymers aus halogeniertem Vinylmonomer je 100 Gewichtsteile Harz umfaßt;

(b) ein Röntgenkontrastmittel hiefür, bestehend aus einer Diiodbenzoat-oder einer Tetraiodbenzoatverbindung der Formel (I) oder aus einem Triiodbenzoat der Formel (II):

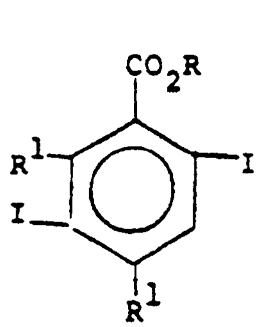 (I)

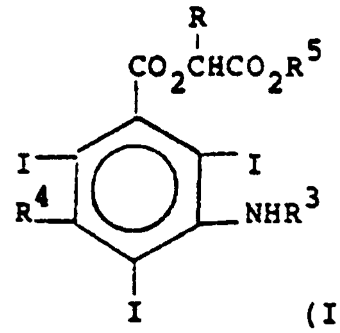 (II),

worin

- R für Alkyl oder Alkyoxyalkyl steht,
- $R^1$ Wasserstoff oder Iod bedeutet,
- $R^2$ Wasserstoff oder Alkyl darstellt,
- $R^3$ für Wasserstoff oder Alkanoyl steht,
- $R^4$ Wasserstoff oder Alkanoylamino bedeutet und
- $R^5$ Alkyl darstellt,

oder aus einem Gemisch dieser Verbindungen, welches Röntgenkontrastmittel 10 bis 40 Gewichtsteile je 100 Gewichtsteile aus (a) und (b) ausmacht, und

(c) (i) eine organische Siliziumverbindung mit wenigstens zwei an Silizium gebundenen ungesättigten Gruppen je Molekül umfaßt; und

der Teil B

(a) ein Harz, welches 100 bis 0 Gewichtsteile eines thermoplastischen Polyurethans und dementsprechend 0 bis 100 Teile eines Polymers aus einem halogenierten Vinylmonomer je 100 Gewichtsteile Harz umfaßt;

(b) ein Röntgenkontrastmittel hiefür, bestehend aus einer Diiodbenzoat-oder einer Tetraiodbenzoatverbindung der Formel

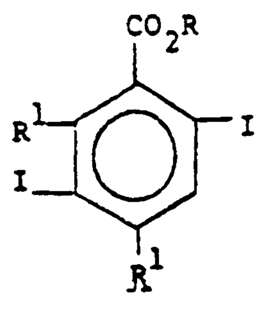 (I)

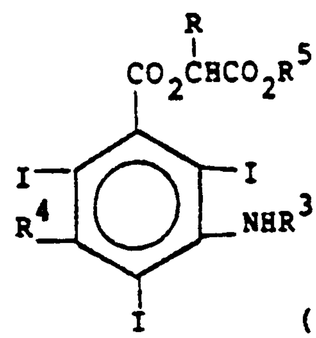 (II),

worin

- R für Alkyl oder Alkoxyalkyl steht,
- $R^1$ Wasserstoff oder Iod bedeutet,
- $R^2$ Wasserstoff oder Alkyl darstellt,
- $R^3$ für Wasserstoff oder Alkanoyl steht,
- $R^4$ Wasserstoff oder Alkanoylamino bedeutet und
- $R^5$ Alkyl darstellt,

oder aus einem Gemisch dieser Verbindungen, welches Röntgenkontrastmittel 10 bis 40 Gewichtsteile je 100 Gewichtsteile aus (a) und (b) ausmacht, und

(c) (ii) eine Siliziumverbindung mit wenigstens zwei an Silizium gebundenen Wasserstoffatomen je Molekül; und

(c) (iii) eine kleine, katalytisch wirksame Menge eines Platin-enthaltenden Katalysators umfaßt, worin die jeweiligen Mengen an (c) (i), (ii) und (iii) so gewählt sind, daß etwa 8 bis etwa 30 Gewichtsteile eines Platin-gehärteten Silikonnetzwerkpolymers nach dem Vermischen und Thermoformen der Teile A und B je 100 Gewichtsteile der Gesamtzusammensetzung erhalten werden, und worin diese zweiteilige Zusammensetzung im wesentlichen frei von Füllstoff ist.

**15.** Zweiteilige Zusammensetzung nach Anspruch 14, worin in dem Röntgenkontrastmittel (b) (I) jeder Rest $R^1$ für Iod steht und R (Nieder)alkyl oder (Nieder)alkoxy(nieder)alkyl bedeutet.

**16.** Zweiteilige Zusammensetzung nach Anspruch 14, worin das Röntgenkontrastmittel (b) (I) und (b) (II) unter n-Butyl-2,3,4,6-tetraiodbenzoat, 2-Ethoxyethyl-2,5-diiodbenzoat, Ethyl(3-amino-2,4,6-triiodbenzoyloxy)acetat, Ethyl-2-(3-amino-2,3,6-triiodbenzoyloxy)butanoat oder einem Gemisch der Vorstehenden ausgewählt ist.

**17.** Zweiteilige Zusammensetzung nach Anspruch 14, die im wesentlichen frei von teilchenförmigem Siliziumdioxid ist.

**18.** Zweiteilige Zusammensetzung nach Anspruch 14, worin die Komponente (c) (i) von Teil A der Formel:

$$(R^6)_a(R^7)_b\ SiO_{\frac{4-a-b}{2}}$$

entspricht;
und die Komponente (c) (ii) von Teil B der Formel:

$$(R^6)_a(H)_b\ SiO_{\frac{4-a-b}{2}}$$

entspricht, wobei

- $R^6$ unter einwertigen, von olefinischer Unsättigung freien Kohlenwasserstoffresten, halogenierten, von olefinischer Unsättigung freien einwertigen Kolenwasserstoffresten, Cyanoalkylresten oder einem Gemisch der vorstehenden Reste ausgewählt ist;
- $R^7$ unter Vinylresten, Allylresten oder einem Gemisch hievon ausgewählt ist;
- a einen Wert von 1,0 bis 2,5 aufweist;
- b einen Wert von 0,005 bis 2,0 aufweist;

und die Summe aus a plus b gleich 1,005 bis 3,0 beträgt.

**19.** Zweiteilige Zusammensetzung nach Anspruch 18, worin die Komponente (c) (iii) unter Chlorplatinsäure, Platinschwarz, auf einem Träger absorbiertem Platin, einem Platin-Olefin-Komplex, einem Platin-Cyclopropan-Komplex oder einem beliebigen Gemisch der vorstehend Genannten ausgewählt ist.

**20.** Medizinisch-chirurgischer Schlauch, umfassend:
(a) ein Harz, welches 100 bis 0 Gewichtsteile eines thermoplastischen Polyurethans und dementsprechend 0 bis 100 Teile eines Polymers eines halogenierten Vinylmonomers je 100 Gewichtsteile Harz umfaßt;
(b) ein Röntgenkontrastmittel hiefür, bestehend aus einer Diiodbenzoat-oder einer Tetraiodbenzoatverbindung der Formel (I) oder eine Triiodbenzoat der Formel (II):

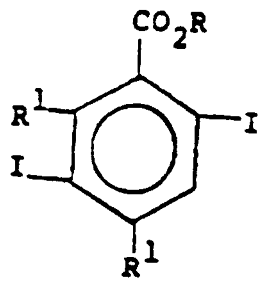
(I)

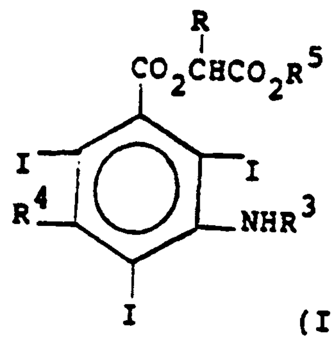
(II)

worin

- $R^1$ Wasserstoff oder lod bedeutet,
- $R^2$ Wasserstoff oder Alkyl darstellt,
- $R^3$ Wasserstoff oder Alkanoyl bedeutet,
- $R^4$ für Wasserstoff oder Alkanoylamino steht und
- $R^5$ Alkyl darstellt,

oder einem Gemisch dieser Verbindungen

welches Röntgenkontrastmittel 10 bis 40 Gewichtsteile je 100 Gewichtsteile von (a) und (b) ausmacht, und, gleichmäßig darin verteilt,

(c) ein Platin-gehärtetes Siliconnetzwerkpolymer, wobei die Menge von (c) 8 bis 30 Gewichtsteile je 100 Gewichtsteile von (a), (b) und (c) ausmacht, worin dieser medizinisch-chirurgische schlauch im wesentlichen frei von Füllstoff ist.

**21.** Medizinisch-chirurgischer Schlauch nach Anspruch 20, worin in dem Röntgenkontrastmittel (b) (I) jeder Rest $R^1$ für lod steht und R (Nieder)alkyl oder (Nieder)alkoxy(nieder)alkyl bedeutet.

**22.** Medizinish-chirugischer Schlauch nach Anspruch 20, worin das Röntgenkontrastmittel (b) (I) n-Butyl-2,3,4,6-tetraiodbenzoat ist.

**23.** Medizinisch-chirurgisher Schlauch nach Anspruch 20, worin das Röntgenkontrastmittel (b) (I) 2-Ethoxyethyl-2,5-diiodbenzoat ist.

**24.** Medizinisch-chirurgischer Schlauch nach Anspruch 20, worin Röntgenkontrastmittel (b) (I) ein Gemisch aus n-Butyl-2,3,4,6-tetraiodbenzoat und 2-Ethoxyethyl-2,5-diiodbenzoat ist.

**25.** Medizinisch-chirurgischer Schlauch nach Anspruch 20, worin das Röntgenkontrastmittel (b) (II) Ethyl-(3-amino-2,4,6-triiodbenzoyloxy)acetat ist.

**26.** Medizinisch-chirurgischer Schlauch nach Anspruch 20, worin das Röntgenkontrastmittel (b) (II) Ethyl-2-(3-amino-2,4,6-triiodbenzoyloxy)butanoat ist.

**27.** Medizinisch-chirurgischer Schlauch nach Anspruch 20, worin die Harzkomponente (a) 100 bis 33 Gewichtsteile thermoplatisches Polyurethane und dementsprechend 0 bis 67 Gewichtsteile eines Polymers eines halogenierten Vinylmonomers je 100 Gewichtsteile Harz umfaßt.

**28.** Medizinisch-chirurgischer Schlauch nach Anspruch 20, worin die Harzkomponente (a) ein Vinylharz unfaßt, das ein Polymer oder Copolymer eines halogenierten Vinylmonomers ist.

**29.** Medizinisch-chirurgischer Schlauch nach Anspruch 20, welcher im wesentlichen frei von teilchenförmigem Siliziumdioxid ist.

**30.** Medizinisch-chirurgischer Schlauch nach Anspruch 20, worin das Platin-gehärtete Siliconnetzwerkpolymer (c) das Reaktionsprodukt aus

(i) einer organischen Siliziumverbindung mit wenigstens zwei an Silizium gebundene ungesättigten Gruppen je Molekül,

(ii) einer Siliziumverbindung mit einem Gehalt an wenigstens zwei an Silizium gebundenen Wasserstoffatomen je Molekül und
(iii) einer kleinen, Katalytisch wirksamen Menge eines Platin-enthaltenden Katalysators umfaßt.

**31.** Medizinisch-chirurgischer Schlauch nach Anspruch 30, worin, die Komponente (i) der Formel:

$$(R^6)_a(R^7)_b\ SiO_{\frac{4-a-b}{2}}$$

entspricht;
und die Komponente (ii) der Formel:

$$(R^6)_a(H)_b\ SiO_{\frac{4-a-b}{2}}$$

entspricht, wobei

- $R^6$ unter einwertigen, von olefinischer Unsättigung freien Kohlenwasserstoffresten, halogenierten, von olefinischer Unsättigung freien einwertigen Kohlenwasserstoffresten, Cyanoalkylresten oder eine Gemisch der vorstehenden Reste ausgewählt ist;
- $R^7$ unter Vinylresten, Allylresten oder eine Gemisch hievon ausgewählt ist;
- a einen Wert von 1,0 bis 2,5 aufweist;
- b einen Wert von 0,005 bis 2,0 aufweist;

und die Summe aus a plus b gleich 1,005 bis 3,0 beträgt.

**32.** Medizinisch-chirurgischer Schlauch nach Anspruch 31, worin die Komponente (iii) unter Chlorplatinsäure, Platinschwarz, auf einem Träger absorbiertem Platin, einem Platin-Olefin-Komplex, einem Platin-Cyclopropan-Komplex oder einem beliebigen Gemisch der vorstehend Genannten ausgewählt ist.

**33.** Medizinisch-chirurgischer Schlauch, umfassend eine Mehrzahl von konzentrischen rohrförmigen Teilen, von denen wenigstens einer eine radioopake Zusammensetzung nach Anspruch 1 umfaßt.

**34.** Medizinisch-chirurgischer Schlauch, umfassend eine Mehrzahl von konzentrischen rohrförmigen Teilen, von denen wenigstens einer eine radioopake Zusammensetzung nach Anspruch 1 umfaßt und von denen wenigstens einer visuell transparent und röntgentransparent ist.

**35.** Verfahren zur Herstellung eines radioopaken medizinisch-chirurgischen Schlauches, welches Verfahren umfaßt:
(1) Zur-Verfügung-Stellen einer zweiteiligen, im wesentlichen von Füllstoff freien Zusammensetzung, worin der Teil A
(a) ein Harz, welches 100 bis 0 Gewichtsteile eines thermoplastischen Polyurethans und dementsprechend von 0 bis 100 Teile eines Polymers aus halogeniertem Vinylmonomer je 100 Gewichtsteile Harz umfaßt;
(b) ein Röntgenkontrastmittel hiefür, bestehend aus einer Diiodbenzoat-oder einer Tetraiodbenzoatverbindung der Formel (I) oder aus einem Triiodbenzoat der Formel (II):

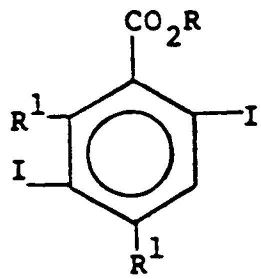 (I) 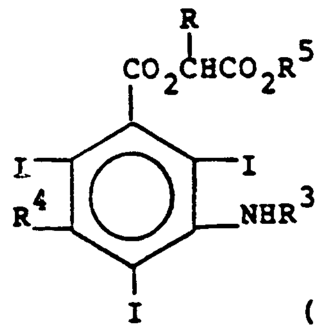 (II),

worin

R für Alkyl oder Alkoxyalkyl steht,
$R^1$ Wasserstoff oder Iod bedeutet,
$R^2$ Wasserstoff oder Alkyl darstellt,
$R^3$ für Wasserstoff oder Alkanoyl steht,
$R^4$ Wasserstoff oder Alkanoylamino bedeutet und
$R^5$ Alkyl darstellt,

oder aus einem Gemisch dieser Verbindungen, welches Röntgenkontrastmittel 10 bis 40 Gewichtsteile je 100 Gewichtsteile aus (a) und (b) ausmacht, und

(c) (i) eine organische Siliziumverbindung mit wenigstens zwei an Silizium gebundenen ungesättigten Gruppen je Molekül umfaßt; und

der Teil B

(a) ein Harz, welches 100 bis 0 Gewichtsteile eines thermoplastischen Polyurethans und dementsprechend 0 bis 100 Teile eines Polymers aus einem halogenierten Vinylmonomer je 100 Gewichtsteile Harz unfaßt;

(b) ein Röntgenkontrastmittel hiefür, bestehend aus einer Diiodbenzoat-oder einer Tetraiodbenzoatverbindung der Formel

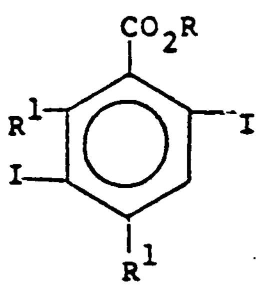 (I) 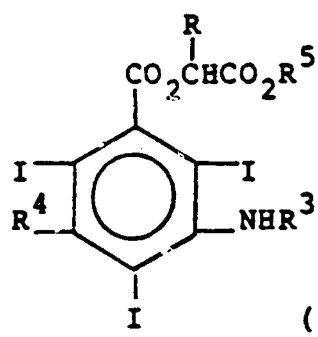 (II),

worin

R für Alkyl oder Alkoxyalkyl steht,
$R^1$ Wasserstoff oder Iod bedeutet,
$R^2$ Wasserstoff oder Alkyl darstellt,
$R^3$ für Wasserstoff oder Alkanoyl steht,
$R^4$ Wasserstoff oder Alkanoylamino bedeutet und
$R^5$ Alkyl darstellt,

oder aus einem Gemisch dieser Verbindungen, welches Röntgenkontrastmittel 10 bis 40 Gewichtsteile je 100 Gewichtsteile aus (a) und (b) ausmacht, und

(c) (ii) eine Siliziumverbindung mit wenigstens zwei an Silizium gebundenen Wasserstoffatomen je Molekül; und

(c) (iii) eine Kleine, katalytisch wirksame Menge eines Platin-enthaltenden Katalysators umfaßt, worin die jeweiligen Mengen an (c) (i), (ii) und (iii) so gewählt sind, daß etwa 8 bis etwa 30 Gewichtsteile eines Platin-gehärteten Silikonnetzwerkpolymers je 100 Gewichtsteile der Gesamtzusammensetzung erhalten werden;

(2) Schmelzmischen von Teil A mit Teil B Und

(3) Extrudieren des Schmelzgemisches von (2) zu Schläuchen bzw. Rohren.

Fig.1

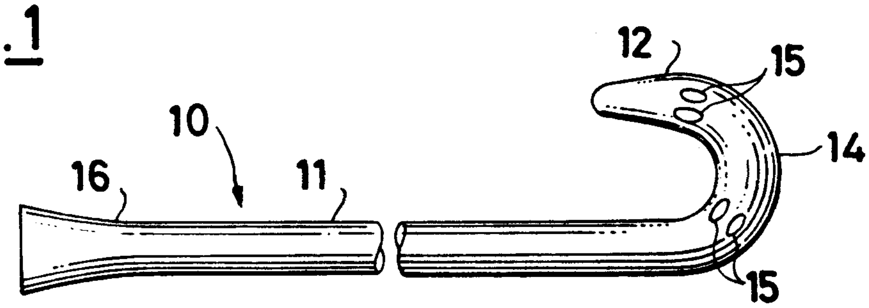


Fig.2

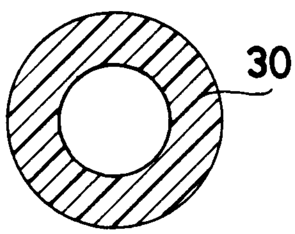


Fig.3

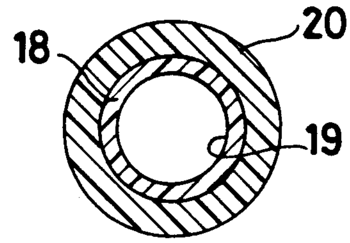


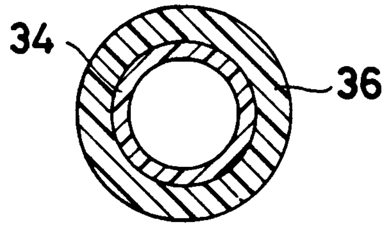


Fig.4

Fig.5

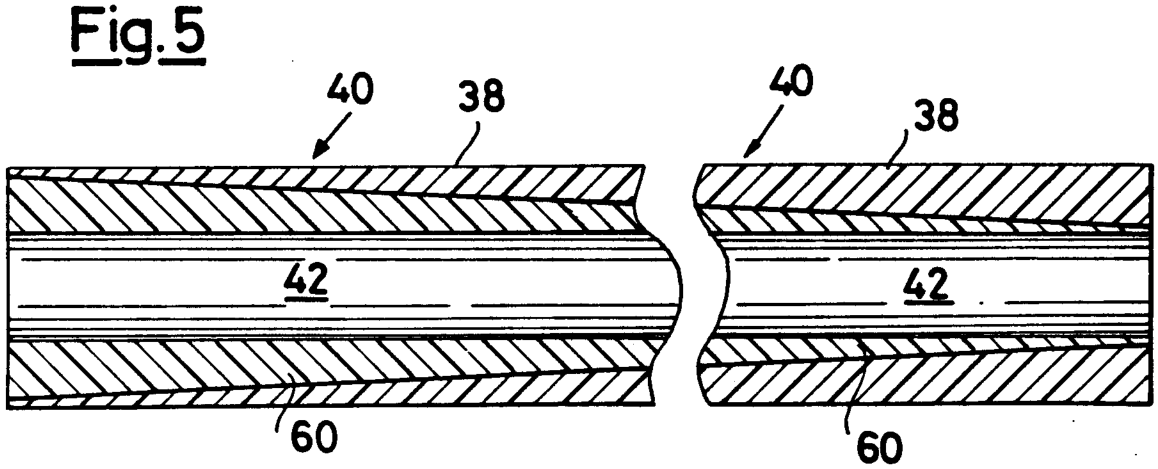

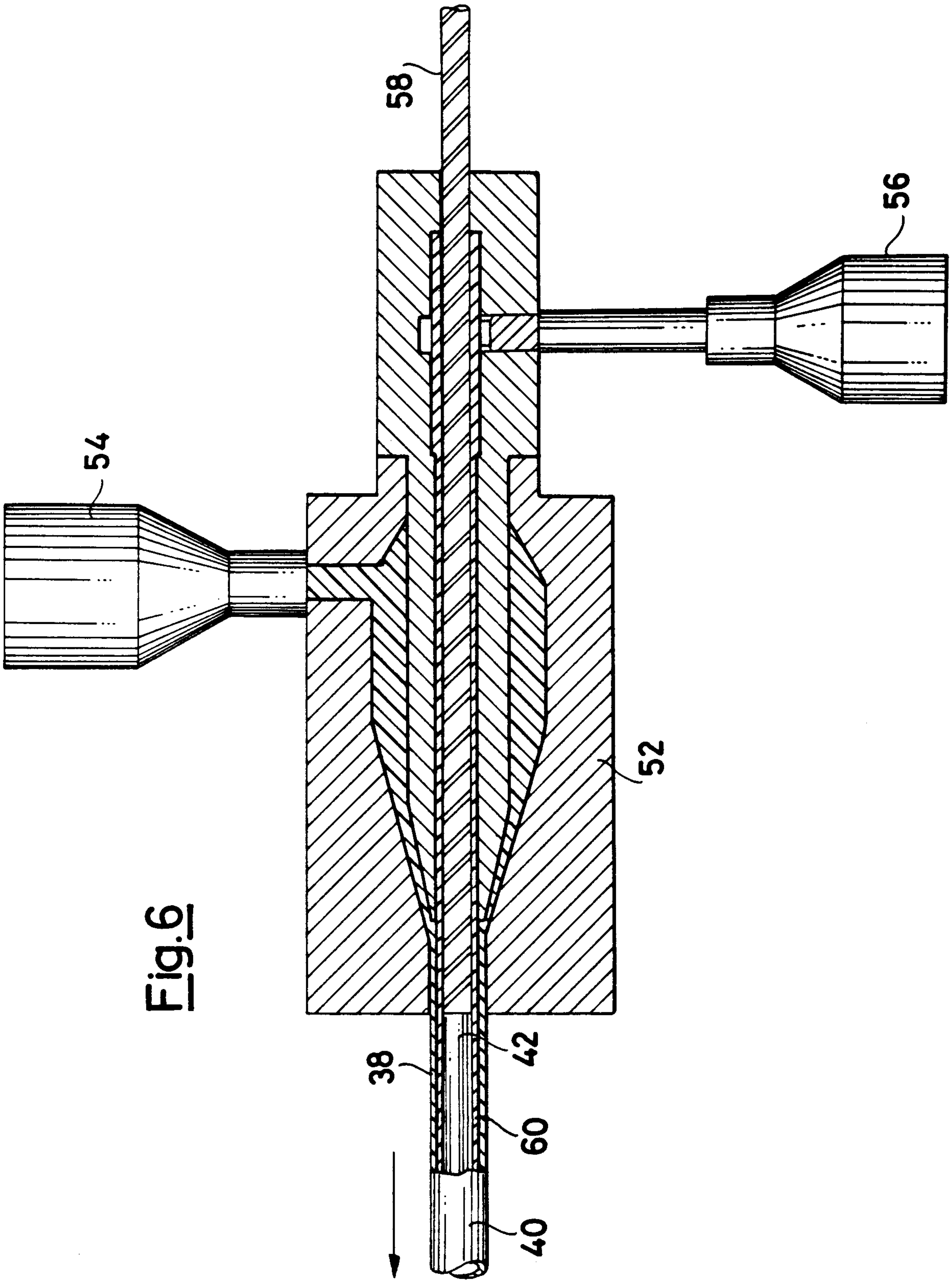


Fig.6